(19) Europäisches Patentamt
European Patent Office
Office européen des brevets

(11) Publication number: **0 138 527 A2**

(12) # EUROPEAN PATENT APPLICATION

(21) Application number: **84306807.3**

(22) Date of filing: **05.10.84**

(51) Int. Cl.⁴: **C 07 D 231/56**, C 07 D 231/54, C 07 D 231/16, C 07 D 231/18, A 01 N 43/56

(30) Priority: **08.10.83 JP 188939/83**
**31.08.84 JP 180365/84**

(43) Date of publication of application: **24.04.85 Bulletin 85/17**

(84) Designated Contracting States: **AT BE CH DE FR GB IT LI NL**

(71) Applicant: **NIPPON KAYAKU KABUSHIKI KAISHA, Tokyo Fujimi Bldg. 11-2 Fujimi 1-chome Chiyoda-ku, Tokyo 102 (JP)**

(72) Inventor: **Yanagi, Mikio, 178-28, Ouaza-Idoki, Ageo-shi Saitama-ken (JP)**
Inventor: **Yamada, Osamu, 473-5 Ouaza-Mukouyama, Ageo-shi Saitama-ken (JP)**
Inventor: **Futatsuya, Fumio, No. 9-306, 710-50 Ouaza-Higashiarai, Omiya-shi Saitama-ken (JP)**
Inventor: **Shida, Atsuhiko, 3-11-6 Higashi-Honcho, Koga-shi Ibaraki-ken (JP)**

(74) Representative: **Woods, Geoffrey Corlett et al, J.A. KEMP & CO. 14 South Square Gray's Inn, London WC1R 5EU (GB)**

(54) **Herbicidal pyrazole derivatives.**

(57) Pyrazole derivatives, for use as herbicides, have the formula (I):

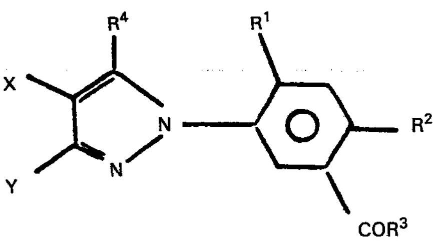

(I)

wherein

$R^1$ represents hydrogen, halogen or methyl;

$R^2$ represents hydrogen, halogen, nitro, methyl, cyano, carboxy, ($C_1$ to $C_8$)-alkoxy or ($C_1$ to $C_4$)-alkoxycarbonyl;

$R^3$ represents hydroxy, amino, ($C_3$ or $C_4$)-alkenyloxy, ($C_3$ or $C_4$)-alkynyloxy, ($C_1$ to $C_4$)-alkoxy-($C_1$ to $C_4$)-alkoxy, ($C_1$ to $C_4$)-alkylthio, ($C_3$ or $C_4$)-alkenylamino, ($C_1$ to $C_4$)-alkylsulfonylamino, ($C_1$ or $C_2$)-alkoxy-($C_1$ or $C_2$)-alkylamino, $-O-N=C-(CH_3)_2$, anilino, benzylamino, morpholino, ($C_1$ to $C_6$)-alkoxy unsubstituted or substituted by ($C_1$ to $C_4$)-alkoxycarbonyl, ($C_1$ to $C_4$)-mono- or di-alkylamino group in which the or each alkyl group is unsubstituted or substituted by hydroxy or carboxy, or -OM wherein M represents an alkali metal, alkaline earth metal, ammonium or ($C_1$ to $C_4$)-alkylammonium;

$R^4$ represents halogen, methyl, ($C_1$ to $C_4$)-alkoxy, $-S(O)_m-R^5$ wherein m is 0, 1 or 2 and $R^5$ represents ($C_1$ to $C_4$)-alkyl;

X represents hydrogen, halogen, ($C_1$ to $C_4$)-alkyl, ($C_1$ to $C_4$)-alkylthio or ($C_1$ to $C_4$)-alkylsulfonyl; and

Y represents ($C_1$ to $C_4$)-alkyl or X and Y taken together with the atoms to which they are attached form a cyclopentyl or cyclohexyl ring unsubstituted or substituted with one or more methyl group.

EP 0 138 527 A2

DESCRIPTION

TITLE: HERBICIDAL PYRAZOLE DERIVATIVES

The present invention relates to pyrazole derivatives, their preparation, herbicidal compositions containing them and their use in controlling the growth of weeds.

It is known that some pyrazole derivatives have herbicidal activity (for example, see Japanese Patent Application Laid-Open No. 52-51365 (1977)). As a result of the present inventors' studies, they have found that certain N-substituted pyrazoles represented by formula (I) show an extremely strong herbicidal activity. The present invention therefore provides derivatives of pyrazole represented by the formula (I):

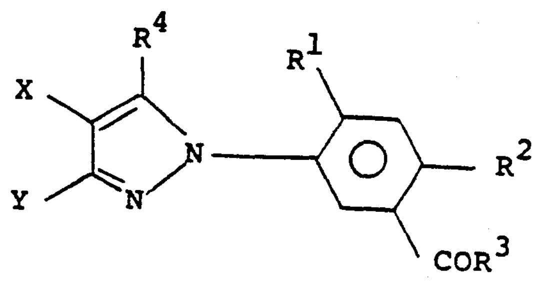

(I)

wherein $R^1$ is hydrogen, halogen or methyl;
$R^2$ is hydrogen, halogen, nitro, cyano, methyl, carboxy, ($C_1$ to $C_4$)-alkoxy or ($C_1$ to $C_4$)-alkoxycarbonyl;
$R^3$ is hydroxy, amino, ($C_3$ or $C_4$)-alkenyloxy, ($C_3$ or $C_4$)-alkynyloxy, ($C_1$to $C_4$)-alkoxy-($C_1$ to $C_4$)-alkoxy, ($C_1$ to $C_4$)-alkylthio, ($C_3$ or $C_4$)-alkenylamino, ($C_1$ to $C_4$)-alkylsulfonylamino, ($C_1$ or $C_2$)-alkoxy-($C_1$ or $C_2$)-alkylamino, $-O-N=C(CH_3)_2$, anilino, benzylamino, morpholino, ($C_1$ to $C_6$)-alkoxy unsubstituted or substituted by ($C_1$ to $C_4$)-alkoxycarbonyl, ($C_1$ to $C_4$) mono- or di-alkylamino in which the or each alkyl group is unsubstituted or substituted by hydroxy or carboxy, or -OM wherein M represents an alkali metal, alkaline

earth metal, ammonium or ($C_1$ to $C_4$)-alkylammonium;

$R^4$ is halogen, methyl, ($C_1$ to $C_4$)-alkoxy or $-S(O)_m-R^5$ wherein m is 0, 1 or 2 and $R^5$ is ($C_1$ to $C_4$)-alkyl;

X is hydrogen, halogen, ($C_1$ to $C_4$)-alkyl, ($C_1$ to $C_4$)-alkylthio or ($C_1$ to $C_4$)-alkylsulfonyl; and

Y is ($C_1$ to $C_4$)-alkyl or X and Y taken together with with the atoms to which they are attached form a cyclopentyl or cyclohexyl ring unsubstituted or substituted by one or more methyl group.

The invention also provides herbicidal compositions containing a compound of formula (I) as active ingredient, together with a suitable adjuvant.

The invention further provides a method of controlling the growth of weeds at a locus, which method comprises applying to the locus a herbicidally effective amount of a compound of formula (I).

The present inventors have found that the pyrazoles of formula (I) can be utilized as a practical herbicide because of their insignificant phytotoxicity to crop plants. The derivatives represented by the formula (I) show an excellent herbicidal activity at a low dosage not only to

the annual weeds in paddy fields such as barnyard grass broad-leaf weeds but also to the perennial weeds in paddy fields such as arrowhead, bulrush, mizugayatsuri, water chestnut and needle spikerush, and also respectively show an excellent herbicidal activity in up-land by both pre- and post emergence treatments, particularly to broad-leaf weeds belonging to the family such as amaranth, goosefoot or buckmeat a low dosage, and that on the other hand the phytotoxicity of the derivative of N-substituted pyrazole represented by the formula (I) is very little to the crop plants such as rice, wheat, oat, maize, soya-bean plant, cotton plant and common sunflower and accordingly, each of the derivatives of N-substituted pyrazole represented by the formula (I) would be a practically useful herbicide.

In the derivatives of N-substituted pyrazole represented by the formula (I) according to the present invention, as the halogen atom, a chlorine atom, a bromine atom, a iodine atom or a fluorine atom may be mentioned, and as the ($C_1$ to $C_8$)-alkoxy group, methoxy group, ethoxy group, n-propoxy group, isopropoxy group, n-butoxy group, sec-butoxy group, tert-butoxy group, n-pentyloxy group, isopentyloxy group, 1,1-dimethylpropoxy group, 1,2-dimethylpropoxy group, n-hexyloxy group, 1-methylpentyloxy group, 1,3-dimethylbutoxy group, 1-ethylbutoxy group, n-heptyloxy group, 1-ethylpentyloxy group, 1-ethylhexyloxy group and 2,2-dimethyl-4-methylpentyloxy group may be mentioned.

As the ($C_3$ to $C_4$)-alkenyloxy group, allyloxy group and allylmethyloxy group may be mentioned. Also, as the ($C_3$ to $C_4$)-alkynyloxy group, propargyloxy group, 2-butynyloxy group and 1-methylpropargyloxy group may be mentioned. As the ($C_1$ to $C_4$)-alkylthio group, methylthio group, ethylthio group, n-propylthio group, isopropylthio group and sec-butylthio group may be mentioned, and as the ($C_3$ to $C_4$)-alkenylamino group, allylamino group, allylmethylamino group may be mentioned. As the ($C_1$ to $C_4$)-alkoxy-($C_1$ to $C_4$)-alkoxy group, methoxymethoxy group, methoxypropoxy group, ethoxypropoxy group, propoxyethoxy group and butoxyethoxy group may be mentioned, and as the ($C_1$ to $C_4$)-alkylsulfonylamino group, methylsulfonylamino group and ethylsulfonylamino group may be mentioned.

As the alkali metal in the OM group, lithium, sodium and potassium, and as the alkaline earth metal in the OM group, calcium, magnesium and barium may be mentioned. As the ($C_1$ to $C_4$)-alkylammonium group in the OM group, methylammonium group, ethylammonium group, propylammonium group, isopropylammonium group, butylammonium group, 2-hydroxyethyl-trimethylammonium group and benzylammonium group may be mentioned.

The novel derivatives of N-substituted pyrazole represented by the formula (I) according to the present invention can be produced, for instance, by the following process:

By reacting an N-substituted pyrazolone represented by the formula (II):

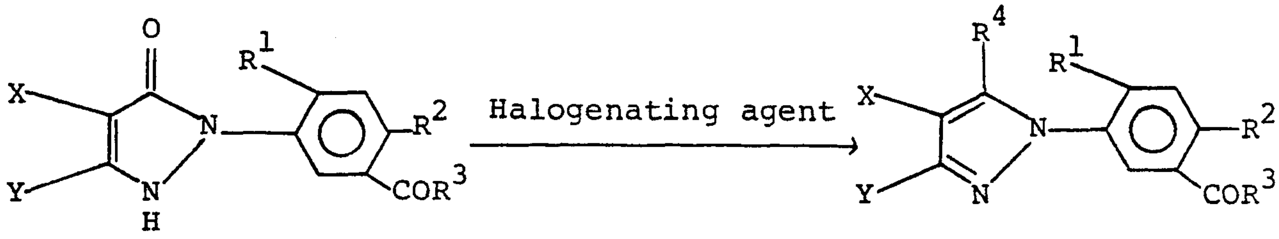


(II) (I)

wherein $R^1$, $R^2$, X and Y are respectively the same as in the formula (I) and $R^3$ represents a hydroxy group, a lower alkoxy group, a lower alkynyloxy group or a lower alkylthio group, with a halogenating agent, preferably at a temperature of from 60 to 180°C for 15 hours with the optional addition of an inert solvent such as chloroform, methylene chloride and toluene, a compound represented by the formula (I) wherein $R^1$, $R^2$, $R^3$, X and Y are respectively the same as in the formula (II) and $R^4$ represents a halogen atom can be obtained. The above-mentioned reaction may be accelerated by the addition of dimethylformamide, pyridine or N,N-dialkylaniline. As the halogenating agent, an ordinary phosphorus chloride, preferably phosphorus oxychloride or phosphorus oxybromide may be used.

By reacting the compound represented by the formula (II) wherein $R^1$, $R^2$, $R^3$, X and Y are respectively the same as in the formula (I) with a dialkyl sulfate represented by the formula (III) wherein $R^5$ represents a lower alkyl group in the presence of a base, for instance, sodium hydroxide and potassium hydroxide and in the presence of an inert solvent such as water, tetrahydrofuran, dioxane, methylene chloride

and toluene, preferably at a temperature of from -20 to 100°C for 1 to 24 hours, a compound represented by the formula (IV):

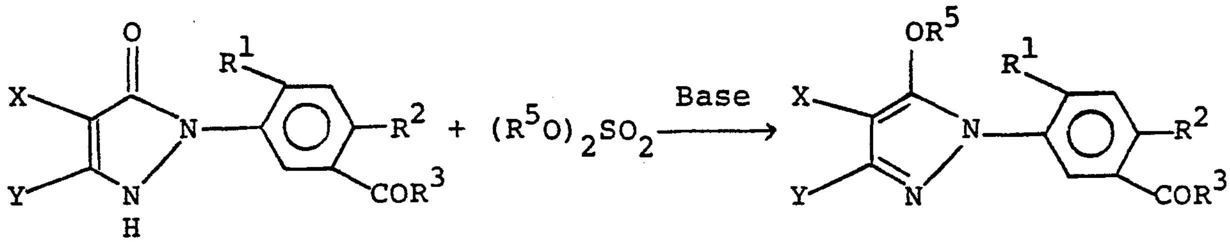


(II) (III) (IV)

wherein $R^1$, $R^2$, $R^3$, $R^5$, X and Y are respectively the same as above, is obtained.

The compound represented by the formula (II) used as a starting material in the above-mentioned syntheses is obtained by bringing a compound represented by the formula (V) wherein $R^6$ is a lower alkyl, and a substituted phenylhydrazine represented by the formula (VI) wherein $R^1$, $R^2$ and $R^3$ are respectively the same as in the formula (I) into dehydration in a suitable solvent, for instance, methylene chloride, an aliphatic- or aromatic hydrocarbon such as toluene, alcohol or ether, preferably at 60 to 150°C for 30 min to 30 hours under a reflux condenser.

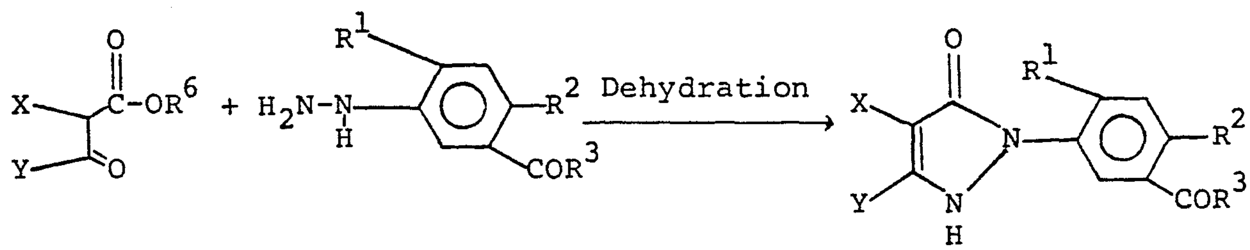


(V) (VI) (II)

In the above-mentioned reaction, if necessary, the reaction can be completed under milder conditions (for example 5°C to temperature of reflux) by the addition of a suitable base, for instance, triethylamine, sodium hydroxide and alcoholates into the reaction system.

The substituted phenylhydrazine represented by the formula (VI) can be obtained by diazotizing a substituted aniline represented by the following formula (VII) wherein $R^1$, $R^2$ and $R^3$ are respectively the same as in the formula (I) with sodium nitrite, and reducing the thus obtained diazonium salt with a reducing agent such as stannous chloride or sodium hydrogen sulfite.

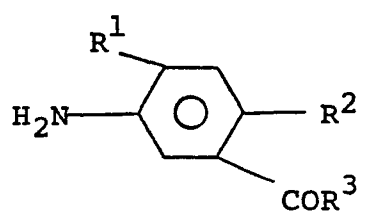

(VII)

The compound represented by the formula (I) wherein $R^4$ is an alkylthio group can be obtained by a process comprising the following steps.

A cycloalkanone represented by the formula (VIII) wherein X and Y are respectively the same as in the formula (I) is reacted with carbon disulfide in the presence of a base following the known method (refer to Tetrahydron Letters, 43, 4207, 1973), thereby obtaining a compound represented by the formula (IX), and the compound is reacted with an alkyl halide represented by the formula (X) or a dialkyl sulfate represented by the formula (III) to obtain a compound

represented by the formula (XI) wherein $R^5$ is the same as above.

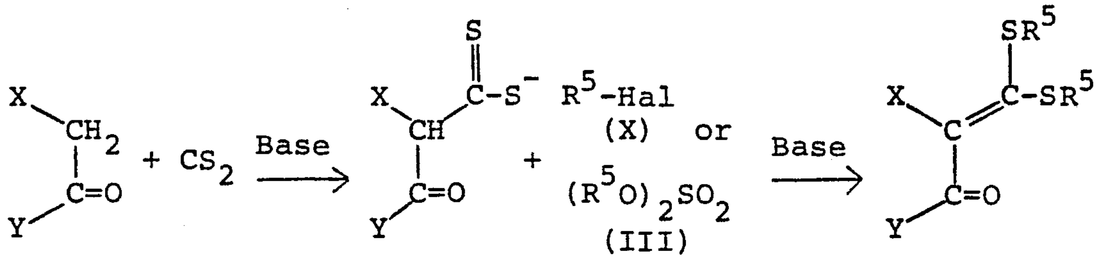


(VIII) (IX) (XI)

Thereafter, the thus obtained compound represented by the formula (XI) is reacted with a substituted phenylhydrazine represented by the formula (VI) wherein $R^1$, $R^2$ and $R^3$ are respectively the same as above, in an inert solvent at a temperature from room temperature to 100°C for 30 min to 5 hours to obtain a compound represented by the formula (XII) wherein $R^1$, $R^2$, $R^3$ and $R^5$ are respectively the same as above. By reacting with the compound represented by the formula (XII) in the presence of an acid catalyst, for instance, hydrogen chloride, while refluxing the mixture at a temperature in a range of from room temperature to the refluxing temperature of the solvent for 1 to 10 hours, a compound represented by the formula (XIII) wherein $R^1$, $R^2$, $R^3$, $R^5$, X and Y are respectively the same as above, is obtained.

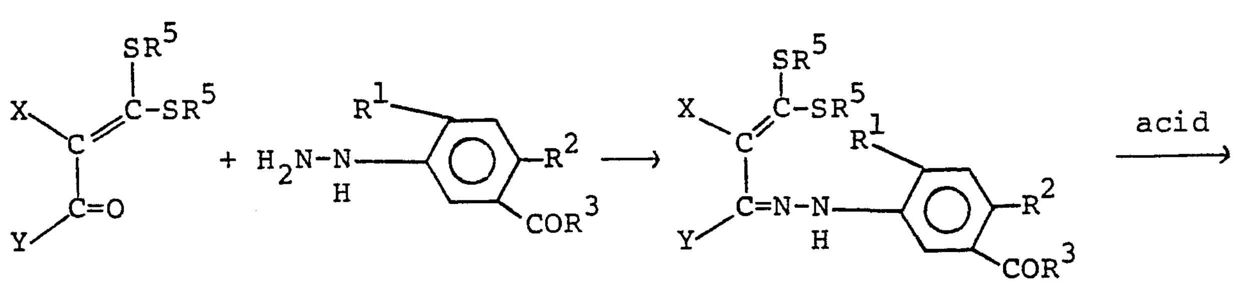


(XI) (VI) (XII)

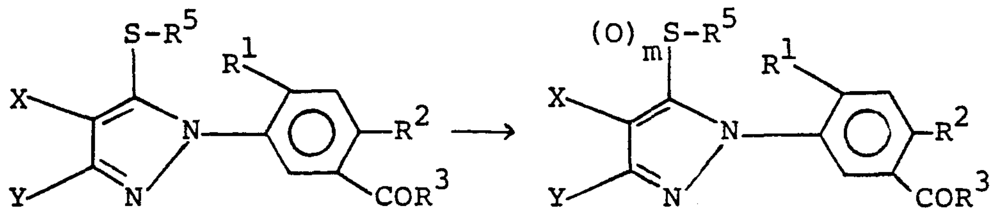

(XIII) (XIV)

By reacting a peroxide such as hydrogen peroxide with the compound represented by the formula (XIII) in an inert solvent at (1) a relatively low temperature of from 0 to 60°C, an alkylsulfinyl derivative represented by the formula (XIV) wherein $R^1$, $R^2$, $R^3$ and $R^5$ are respectively the same as above and m is 1, or at (2) a relatively high temperature of from room temperature to 150°C, an alkylsulfonyl derivative represented by the same formula (XIV) wherein $R^1$, $R^2$, $R^3$ and $R^5$ are respectively the same as above and m is 2 can be synthesized. Since the above-mentioned reaction proceeds to give the alkylsulfonyl derivative via the alkylsulfinyl derivative, it is desirable to check the proceeding state of the reaction by the use of, for instance, a thin-layer chromatography for determining the end point of the reaction in order to obtain both the products respectively.

Further, by reacting a halogenating agent such as thionyl chloride, phosphorus oxichloride and phosphorus oxibromide with a compound represented by the formula (XV) wherein $R^1$, $R^2$, $R^4$, X and Y are respectively the same as in the formula (I), in an inert solvent at a temperature from room temperature to 110°C, thereby obtaining a compound represented by the formula(XVI) wherein $R^1$, $R^2$, $R^4$, X and Y are respectively

the same as in the formula(XV), and by further reacting the thus obtained compound with a compound represented by the formula (XVII):

$$H - R^3 \qquad (XVII)$$

wherein $R^3$ is the same as in the formula (I) except for the case where $R^3$ is a group OM wherein M is an alkali metal atom, in an inert solvent and in the preferred presence of a base such as pyridine at a temperature of from -5 to 100°C, a compound represented by the formula (I) can be obtained.

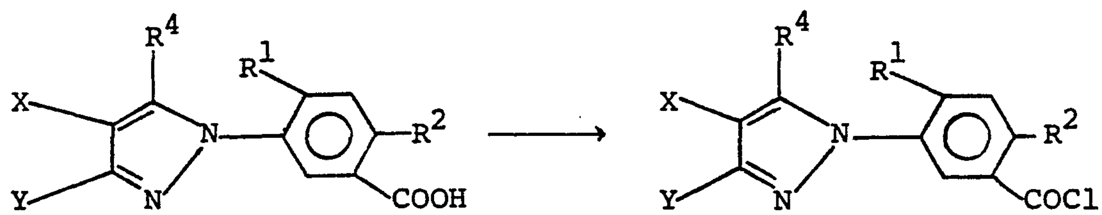

(XV) (XVI)

In the case of producing the compound represented by the formula (I) wherein X is a halogen atom, a compound represented by the formula (XVIII) wherein $R^1$, $R^2$, $R^3$, $R^4$ and Y are respectively the same as in the formula (I) is subjected to halogenation in an inert solvent such as acetic acid and chloroform at a preferable temperature of 0 to 30°C by chlorine or bromine, or is reacted with iodine and mercuric oxide at a preferable temperature of 50 to 100°C in an inert solvent such as benzene to obtain the object compound represented by the

formula (I) wherein $R^1$, $R^2$, $R^3$, $R^4$ and Y is respectively the same as in the formula (XVIII) and X is a halogen atom.

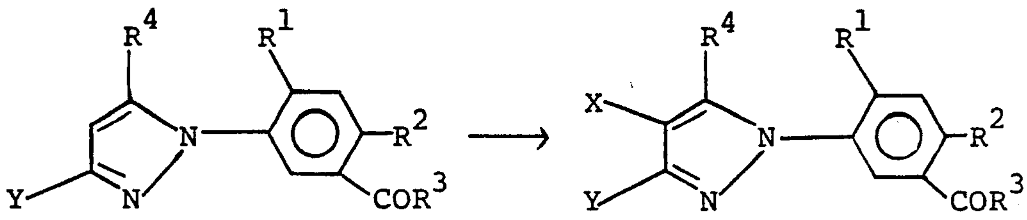

(XVIII)                    (I)

The compound represented by the formula (I) wherein $R^3$ is a OM group wherein M is an alkali metal atom is obtained by reacting a compound represented by the formula (XV) with sodium hydroxide, sodium carbonate, potassium hydroxide or potassium carbonate, preferably in an aqueous solution following a conventional method.

As the inert solvent according to the present invention, an aromatic hydrocarbon such as benzene, toluene and xylene and a halogen derivative thereof such as chlorobenzene, an aliphatic hydrocarbon such as n-hexane, n-heptane and petroleum ether, a cycloaliphatic hydrocarbon such as cyclohexane, a halogenated aliphatic hydrocarbon such as chloroform, carbon tetrachloride and tetrachloroethylene, a ketone such as acetone and ethyl methyl ketone, an ether such as ethyl ether, tetrahydrofuran and dioxane, an alcohol such as methanol and ethanol, an ester such as ethyl acetate, an amide such as dimethylformamide and water may be mentioned.

The present invention is further illustrated by the following examples.

SYNTHETIC EXAMPLE 1:

Production of 3-chloro-2-(4-chloro-3-isopropoxycarbonylphenyl)-4,5,6,7-tetrahydro-2H-indazole (compound No.2)

Into 9.25 g (0.028 mol) of 2-(4-chloro-3-isopropoxycarbonyl)-1,2,4,5,6,7-hexahydro-3H-indazole-3-one, 3 ml of phosphorus oxychloride were added, and after stirring the mixture well, 4.7 ml of N,N-diethylaniline were slowly added to the mixture and the whole mixture was heated for 5 hours at 130 to 140°C. After cooling the reaction mixture and extracting with chloroform, the extract was purified by a silicagel column to obtain 8.5 g of a pale yellow oil showing [$n_D^{25}$] of 1.5741 in a yield of 87.4 %. The product gave the following data of elementary analysis:

| | | C(%) | H(%) | N(%) |
|---|---|---|---|---|
| Found | : | 57.87 | 5.16 | 7.99 |
| Calcd. as $C_{17}H_{18}Cl_2N_2O_2$ | : | 57.80 | 5.14 | 7.93 |

The starting material of the object compound, 2-(4-chloro-3-isopropyloxycarbonyl)-1,2,4,5,6,7-hexahydro-3H-indazole-3-one, was produced as follows.

Into 200 ml of toluene, 20.0 g (0.0087 mol) of 4-chloro-3-isopropoxycarbonylphenylhydrazine and 14.1 g (0.083 mol) of 2-carboethoxycyclohexanone were added, and after stirring the mixture, it was heated under reflux for 6 hours, and the reaction mixture was concentrated by an evaporator. The thus formed crystals were filtered and washed with a small amount of toluene to obtain 16 g of white crystals having a melting point of 177 to 178°C in a yield of 59.3 %. The thus obtained crystals gave the following data of elementary analysis:

| | C(%) | H(%) | N(%) |
|---|---|---|---|
| Found : | 61.20 | 5.74 | 8.41 |
| Calcd. as $C_{17}H_{19}Cl_1N_2O_3$ : | 60.99 | 5.72 | 8.37 |

SYNTHETIC EXAMPLE 2:

Production of 3-chloro-2-(4-chloro-3-carboxyphenyl)-4,5,6,7-tetrahydro-2H-indazole (compound No. 39)

Into a mixture of 8.05 g (0.023 mol) of 3-chloro-2-(4-chloro-3-isopropoxycarbonylphenyl)-4,5,6,7-tetrahydro-2H-indazole and 50 ml of ethanol, an aqueous solution of 1.5 g (0.037 mol) of sodium hydroxide in 5 ml of water was added, and after heating the mixture to 40°C on a warm water bath and stirring for 5 min, the reaction mixture was acidified with a dilute hydrochloric acid. The crystals formed by adding 100 ml of water to the acidified reaction mixture were collected by filtration and dried to obtain 6.16 g of white crystals having a melting point of 189 to 190°C in a yield of 93.0 %. The thus

obtained object compound gave the following data of elementary analysis:

| | C(%) | H(%) | N(%) |
|---|---|---|---|
| Found : | 54.01 | 3.92 | 9.10 |
| Calcd. as $C_{14}H_{12}Cl_2N_2O_2$ : | 54.04 | 3.89 | 9.00 |

SYNTHETIC EXAMPLE 3:

Production of 3-chloro-2-(4-chloro-2-fluoro-5-isopropoxycarbonylphenyl)-4,5,6,7-tetrahydro-5-methyl-2H-indazole (Compound No. 31)

Into 13.45 g (0.037 mol) of 2-(4-chloro-2-fluoro-5-isopropoxycarbonylphenyl)-1,2,4,5,6,7-hexahydro-5-methyl-3H-indazole-3-one, 4.0 ml of phosphorus oxychloride were added, and after stirring the mixture, 6.3 ml of N,N-dimethylaniline were added to the mixture and after heating the mixture at 130 to 140°C for 4 hours under stirring, the reaction mixture was cooled and extracted with ethyl acetate. After washing the extract with an aqueous dilute solution of hydrochloric acid, an aqueous solution of sodium hydrogen carbonate and water, the washed extract was dried over anhydrous sodium sulfate and purified by a silica gel column to obtain 5.5 g of white crystals having a melting point of 65 to 66°C in a yield of 39.1 %. The thus obtained object compound gave the following data of elementary analysis:

| | C(%) | H(%) | N(%) |
|---|---|---|---|
| Found : | 56.04 | 4.91 | 7.35 |
| Calcd. as $C_{18}H_{19}Cl_2FN_2O_2$ : | 56.12 | 4.97 | 7.27 |

SYNTHETIC EXAMPLE 4:

Production of 2-(4-chloro-2-fluoro-5-isopropoxyphenyl)-1,2,4,5,6,7-hexahydro-5-methyl-3H-indazole-3-one

Into 100 ml of toluene, 29.3 g (0.12 mol) of 4-chloro-2-fluoro-3-isopropoxycarbonylphenylhydrazine and 22.8 g (0.12 mol) of 4-methyl-2-carboethoxycyclohexanone were added, and after stirring the mixture, it was heated for 3 hours under reflux. The crystals formed by concentrating the reaction mixture were collected by filtration and washed with a small amount of ether to obtain 16.6 g of white crystals having a melting point of 191 to 192°C in a yield of 38 %. The thus obtained object product gave the following data of elementary analysis:

| | | C(%) | H(%) | N(%) |
|---|---|---|---|---|
| Found | : | 58.89 | 5.53 | 7.70 |
| Calcd. as $C_{18}H_{20}ClFN_2O_3$ | : | 58.94 | 5.50 | 7.64 |

SYNTHETIC EXAMPLE 5:

Production of 2-(4-chloro-2-fluoro-5-isopropoxycarbonylphenyl)-4,5,6,7-tetrahydro-5-methyl-3-methoxy-2H-indazole (Compound No. 32)

Into 160 ml of tetrahydrofuran, 18.34 g (0.05 ml)

of 2-(4-chloro-2-fluoro-5-isopropoxycarbonylphenyl)-1,2,4,5,6,7-hexahydro-5-methyl-3H-indazole-3-one were dissolved, and after adding 6.6 ml of dimethyl sulfate to the solution, 34.0 g of an aqueous 10 % solution of sodium hydroxide were slowly added to the mixture. After stirring the whole mixture for 2 hours, the mixture was extracted with ether, and after washing the extract to neutral with water and drying the thus washed extract over anhydrous sodium sulfate, the dried extract was purified by silica gel column to obtain 1.33 g of the object compound as a yellow oil showing [$n_D^{25}$] of 1.5512 in a yield of 7.0 %. The product gave the following data of elementary analysis:

| | | C(%) | H(%) | N(%) |
|---|---|---|---|---|
| Found | : | 59.84 | 5.87 | 7.40 |
| Calcd. as $C_{19}H_{22}ClFN_2O_3$ | : | 59.92 | 5.82 | 7.35 |

SYNTHETIC EXAMPLE 6:

Production of 3-chloro-2-(4-chloro-2-fluoro-5-isopropoxycarbonylphenyl)-4,5,6,7-tetrahydro-2H-indazole (Compound No. 9)

To 6 g (0.017 mol) of 2-(4-chloro-2-fluoro-5-isopropoxycarbonylphenyl)-1,2,4,5,6,7-hexahydro-3H-indazole-3-one, 1.85 ml of phosphorus oxychloride were added and into the thus prepared mixture, 2.9 ml of N,N-dimethylaniline were slowly added. After heating the mixture for 5 hours at 130 to 140°C while stirring, the reaction mixture was cooled to room

temperature and extracted with ethyl acetate. After washing the extract with an aqueous dilute hydrochloric acid, aqueous dilute solution of sodium hydrogen carbonate and water, the thus washed extract was dried over anhydrous sodium sulfate and after condensing, the concentrate was purified by silica gel column to obtain 3.5 g of a pale yellow oil showing $[n]_D^{25}$ of 1.5578 in a yield of 55.6 %. The thus obtained product gave the following data of elementary analysis:

| | | C(%) | H(%) | N(%) |
|---|---|---|---|---|
| Found | : | 55.22 | 4.65 | 7.60 |
| Calcd. as $C_{17}H_{17}Cl_2FN_2O_2$ | : | 55.00 | 4.62 | 7.54 |

SYNTHETIC EXAMPLE 7:

Production of 2-(4-bromo-2-fluoro-5-isopropoxycarbonylphenyl)-4,5,6,7-tetrahydro-3-methoxy-2H-indazole (Compound No. 26)

Into 200 ml of tetrahydrofuran, 33 g (0.083 mol) of 2-(4-bromo-2-fluoro-5-isopropoxycarbonylphenyl)-1,2,4,5,6,7-hexahydro-3H-indazole-3-one were dissolved, and after adding 10.8 ml of dimethyl sulfate to the solution, and while keeping the temperature of the mixture at 7 to 9°C, 55.6 g of aqueous 10 % by weight solution of sodium hydroxide were added to the mixture.

Thereafter, the mixture was stirred for 2 hours at 7 to 9°C, and the mixture was extracted with ether, and after washing the extract until the washing turned to neutral, the neuralized extract was dried on anhydrous sodium sulfate,

concentrated and purified by silica gel column to obtain 5.5 g of pale brown crystals having a melting point of 83 to 84°C in a yield of 16.1 %.

The thus obtained object product showed the following data of elementary analysis:

| | | C(%) | H(%) | N(%) |
|---|---|---|---|---|
| Found | : | 52.44 | 4.93 | 6.90 |
| Calcd. as $C_{18}H_{20}BrFN_2O_3$ | : | 52.57 | 4.90 | 6.81 |

SYNTHETIC EXAMPLE 8:

Production of 2-(4-chloro-2-fluoro-5-isopropoxycarbonylphenyl)-4,5,6,7-tetrahydro-3-methylthio-2H-indazole (Compound No. 12)

A mixture of 36.4 g (0.145 mol) of 4-chloro-6-fluoro-5-isopropoxycarbonylphenylhydrazine, 24 g (0.12 mol) of α-bis-(methylthio)-methylenecyclohexanone and 200 ml of isopropyl alcohol was heated under reflux while stirring thereof for 3 hours. After adding 100 ml of water and 20 ml of concentrated hydrochloric acid to the mixture, the mixture was stirred for 3 hours at 70°C. After cooling the reaction mixture to room temperature, the reaction mixture was extracted with ethyl acetate, and after washing the extract until it became neutral, the extract was dried over anhydrous sodium sulfate, concentrated and purified by a column-chromatography to obtain 29.3 g of the object product as a brown oil showing [$n_D^{25}$] of 1.5785 in a yield of 65 %. The thus obtained product gave the following data of elementary analysis:

| | | C(%) | H(%) | N(%) |
|---|---|---|---|---|
| Found | : | 56.61 | 5.30 | 7.51 |
| Calcd. as $C_{18}H_{20}ClFN_2O_2S$ | : | 56.47 | 5.27 | 7.32 |

SYNTHETIC EXAMPLE 9:

Production of 2-(4-chloro-2-fluoro-5-isopropoxycarbonylphenyl)-4,5,6,7-tetrahydro-3-methylsulfinyl-2H-indazole (Compound No.51)

Into 100 ml of acetic acid, 5.5 g of 2-(4-chloro-2-fluoro-5-isopropoxycarbonylphenyl)-4,5,6,7-tetrahydro-3-methylthio-2H-indazole were dissolved, and 2.0 g of aqueous 35 % solution of hydrogen peroxide were added to the solution, and the mixture was heated for 2 hours at 50°C under stirring. After cooling the reaction mixture to room temperature, the reaction mixture was poured into a large amount of ice water and extracted with ethyl acetate.

By purifying the extract in a silica gel column-chromatography, 5.3 g of the object compound were obtained as yellow crystals having a melting point of 105 to 107°C in a yield of 92.7 %. The product gave the following data of elementary analysis:

| | | C(%) | H(%) | N(%) |
|---|---|---|---|---|
| Found | : | 54.32 | 5.11 | 7.12 |
| Calcd. as $C_{18}H_{20}ClFN_2O_3S$ | : | 54.20 | 5.05 | 7.02 |

SYNTHETIC EXAMPLE 10:

Production of 2-(4-chloro-2-fluoro-5-isopropoxycarbonylphenyl)-4,5,6,7-tetrahydro-3-methylsulfonyl-2H-indazole (Compound No. 52)

Into 100 ml of acetic acid, 5.5 g of 2-(4-chloro-2-fluoro-5-isopropoxycarbonylphenyl)-4,5,6,7-tetrahydro-3-methylthio-2H-indazole were dissolved, and 4.2 g of aqueous 35 % solution of hydrogen peroxide were added to the solution, and the mixture was stirred for 4 hours at 70°C under heating. After cooling the reaction mixture to room temperature, the reaction mixture was poured into a large amount of ice water, and was extracted with ethyl acetate. After washing the extract with water until it became neutral, the extract was dried over anhydrous sodium sulfate and concentrated. By purifying the concentrate with a silica gel column, 5.7 g of the object compound were obtained as pale yellow crystals having a melting point of 130 to 133°C in a yield of 95.8 %. The object product gave the following data of elementary analysis:

| | C(%) | H(%) | N(%) |
|---|---|---|---|
| Found | : 52.23 | 4.81 | 6.80 |
| Calcd. as $C_{18}H_{20}ClFN_2O_4S$ | : 52.11 | 4.86 | 6.75 |

SYNTHETIC EXAMPLE 11:

Production of 3-chloro-2-(4-chloro-3-methoxycarbonylphenyl)-4,5,6,7-tetrahydro-2H-indazole (Compound No.7)

A mixture of 4.1 g (0.013 mol) of 3-chloro-2-(4-chloro-3-carboxyphenyl)-4,5,6,7-tetrahydro-2H-indazole, 1.8 ml of thionyl chloride and 20 ml of chloroform was heated for 3 hours under reflux, and thereafter, the solvent was distilled off from the reaction mixture. After adding 5 ml of methanol into the distillation residue, 2.1 ml of triethylamine were slowly added to the mixture, and the mixture was extracted with ethyl acetate. After washing the extract with water until the washing became neutral, the washed extract was dried over anhydrous sodium sulfate, concentrated and purified by a silica gel column to obtain 4.1 g of the object compound as white crystals having a melting point of 93 to 94°C in a yield of 97 %. The thus obtained compound gave the following data of elementary analysis:

| | C(%) | H(%) | N(%) |
|---|---|---|---|
| Found : | 55.45 | 4.38 | 8.70 |
| Calcd. as $C_{15}H_{14}Cl_2N_2O_2$: | 55.40 | 4.34 | 8.61 |

SYNTHETIC EXAMPLE 12:

Production of 3-chloro-2-(4-chloro-2-fluoro-5-allylaminocarbonylphenyl)-4,5,6,7-tetrahydro-2H-indazole (Compound No. 38)

A mixture of 4.2 g (0.012 mol) of 3-chloro-2-(4-chloro-2-fluoro-5-chlorocarbonylphenyl)-4,5,6,7-tetrahydro-2H-indazole and 100 ml of toluene was cooled, and while stirring thereof, 1.64 g of allylamine were added thereto. Then the mixture was extracted by ethyl acetate, and after washing the

extract with water until the washing became neutral, the extract was purified by a silica gel column to obtain 3.6 g of the object compound as a colourless oil showing [$n_D^{25}$] of 1.5582 in a yield of 90 %. The thus obtained compound gave the following data of elementary analysis:

| | | C(%) | H(%) | N(%) |
|---|---|---|---|---|
| Found | : | 55.51 | 4.40 | 11.53 |
| Calcd. as $C_{17}H_{16}Cl_2FN_3O$ | : | 55.45 | 4.38 | 11.41 |

SYNTHETIC EXAMPLE 13:

Production of 3-chloro-2-(4-bromo-2-fluoro-5-ethylthiocarbonylphenyl)-4,5,6,7,-tetrahydro-2H-indazole (Compound No. 30)

A mixture of 4.6 g (0.0123 mol) of 3-chloro-2-(4-bromo-2-fluoro-5-carboxyphenyl)-4,5,6,7-tetrahydro-2H-indazole, 1.8 ml of thionyl chloride and 20 ml of chloroform was heated under reflux, and after distilling the solvent off from the mixture, 50 ml of toluene were added to the residue and it was cooled.

After adding 1.2 ml of ethylmercaptane to the cooled mixture, 2.1 ml of triethylamine were slowly added to the mixture while stirring thereof and the reaction mixture was extracted with ethyl acetate. The extract was washed with water until the washing became neutral, dried over anhydrous sodium sulfate and purified by a silica gel column to obtain 4.8 g of the object product as a colorless oil showing

$[n_D^{25}]$ of 1.6100 in a yield of 93.4 %. The thus obtained compound gave the following data of elementary analysis:

| | | C(%) | H(%) | N(%) |
|---|---|---|---|---|
| Found | : | 46.07 | 3.68 | 6.92 |
| Calcd. as $C_{16}H_{15}BrClFN_2OS$ | : | 46.01 | 3.62 | 6.71 |

The present compounds represented by the formula (Ia) and obtained by one of the above-mentioned methods are summarized in the following Table 1:

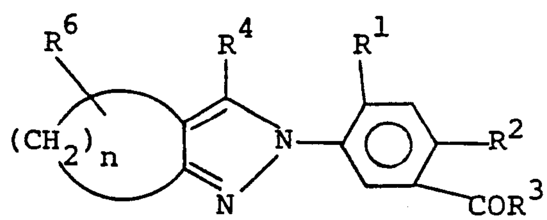

(Ia)

Table 1

| Compound Number | Substituent $R^1$ | $R^2$ | $R^3$ | $R^4$ | $R^6$ | n | Melting point (°C) or (Refractive index $[n_D^{25}]$) | Appearance |
|---|---|---|---|---|---|---|---|---|
| 1 | H | Cl | $C_2H_5O$ | Cl | H | 4 | 42 - 43°C | white crystal |
| 2 | H | Cl | $i\text{-}C_3H_7O$ | Cl | H | 4 | (1.5741) | pale yellow oil |
| 3 | H | Cl | $i\text{-}C_3H_7O$ | $CH_3O$ | H | 4 | 81 - 85°C | white crystal |
| 4 | H | Cl | $i\text{-}C_3H_7O$ | Cl | H | 3 | (1.5775) | pale green oil |
| 5 | H | Br | $i\text{-}C_3H_7O$ | Cl | H | 4 | (1.5820) | pale green oil |
| 6 | H | Br | $i\text{-}C_3H_7O$ | $CH_3O$ | H | 4 | 74 - 77°C | white crystal |
| 7 | H | Cl | $CH_3O$ | Cl | H | 4 | 93 - 94°C | white crystal |
| 8 | F | Cl | $i\text{-}C_3H_7O$ | $CH_3O$ | H | 4 | (1.5492) | yellow oil |
| 9 | F | Cl | $i\text{-}C_3H_7O$ | Cl | H | 4 | (1.5578) | pale yellow oil |
| 10 | F | Cl | $C_2H_5O$ | Cl | H | 4 | (1.5660) | pale yellow oil |
| 11 | F | Cl | $C_2H_5O$ | $CH_3S$ | H | 4 | (1.5684) | light brown oil |
| 12 | F | Cl | $i\text{-}C_3H_7O$ | $CH_3S$ | H | 4 | (1.5785) | light brown oil |
| 13 | F | Cl | HO | Cl | H | 4 | 219 - 222°C | white crystal |
| 14 | F | Cl | $CH_3O$ | Cl | H | 4 | 95 - 97°C | white crystal |
| 15 | F | Cl | $Na^+O^-$ | Cl | H | 4 | >290°C | white crystal |

Table 1 (continued)

| | | | | | | | | |
|---|---|---|---|---|---|---|---|---|
| 16 | H | Cl | $K^{+}O^{-}$ | Cl | H | 4 | 238 - 240°C | white crystal |
| 17 | H | Cl | $(Ca^{2+})_{1/2}O^{-}$ | Cl | H | 4 | 230°C | white crystal |
| 18 | H | Cl | $NH_4^{+}O^{-}$ | Cl | H | 4 | 123 - 125°C | white crystal |
| 19 | H | Cl | $C_2H_5N^{+}H_3O$ | Cl | H | 4 | 108 - 111°C | white crystal |
| 20 | F | Cl | s-$C_4H_9O$ | Cl | H | 4 | (1.5514) | pale yellow oil |
| 21 | F | Cl | s-$C_4H_9O$ | $CH_3O$ | H | 4 | (1.5372) | pale yellow oil |
| 22 | F | Cl | $CH_3$-$CH(CH_3)$-<br>$CH_2$-$CH(CH_3)$-O | Cl | H | 4 | (1.5448) | colourless oil |
| 23 | F | Br | HO | Cl | H | 4 | 222 - 225°C | white crystal |
| 24 | F | Br | n-$C_3H_7O$ | Cl | H | 4 | 74 - 75.5°C | white crystal |
| 25 | F | Br | i-$C_3H_7O$ | Cl | H | 4 | (1.5702) | pale yellow oil |
| 26 | F | Br | i-$C_3H_7O$ | $CH_3O$ | H | 4 | 83 - 84°C | pale brown oil |
| 27 | F | Br | s-$C_4H_9O$ | Cl | H | 4 | (1.5596) | pale yellow oil |
| 28 | F | Br | s-$C_4H_9O$ | $CH_3O$ | H | 4 | (1.5522) | pale orange oil |
| 29 | F | Cl | $C_2H_5S$ | Cl | H | 4 | (1.5973) | colourless oil |
| 30 | F | Br | $C_2H_5S$ | Cl | H | 4 | (1.6100) | colourless oil |
| 31 | F | Cl | i-$C_3H_7O$ | Cl | 5-$CH_3$ | 4 | 65 - 66°C | white crystal |

Table 1 (Continued)

| | | | | | | | | |
|---|---|---|---|---|---|---|---|---|
| 32 | F | Cl | i-$C_3H_7O$ | $CH_3O$ | 5-$CH_3$ | 4 | (1.5512) | yellow oil |
| 33 | F | Cl | s-$C_4H_9O$ | Cl | 5-$CH_3$ | 4 | (1.5500) | pale yellow oil |
| 34 | F | Br | i-$C_3H_7O$ | Cl | 5-$CH_3$ | 4 | 63 - 65°C | white crystal |
| 35 | F | Cl | HO | Cl | 5-$CH_3$ | 4 | 207 - 208°C | white crystal |
| 36 | F | CN | i-$C_3H_7O$ | Cl | H | 4 | 94 - 96°C | white crystal |
| 37 | F | CN | n-$C_3H_7O$ | Cl | H | 4 | 92 - 94°C | white crystal |
| 38 | F | Cl | $CH_2$=$CHCH_2NH$ | Cl | H | 4 | (1.5582) | colourless oil |
| 39 | H | Cl | HO | Cl | H | 4 | 189 - 190°C | white crystal |
| 40 | F | Cl | HO | $CH_3S$ | H | 4 | 185 - 186°C | pale brown crystal |
| 41 | F | Cl | $CH_3O$ | $CH_3S$ | H | 4 | 114 - 115°C | pale yellow crystal |
| 42 | F | Cl | $CH_3O$ | Cl | 5-$CH_3$ | 4 | 68 - 69°C | white crystal |
| 43 | F | Cl | $CH\equiv CCH_2O$ | Cl | 5-$CH_3$ | 4 | 96 - 98°C | pale brown crystal |
| 44 | H | Br | HO | Cl | H | 4 | 214 - 216°C | white crystal |
| 45 | H | Br | $CH_3O$ | Cl | H | 4 | 72 - 74°C | pale yellow crystal |
| 46 | H | Br | i-$C_3H_7O$ | Cl | H | 4 | (1.5860) | pale yellow oil |
| 47 | F | Cl | $NH_2$ | Cl | H | 4 | 209 - 210°C | white crystal |

Table 1 (continued)

| | | | | | | | | |
|---|---|---|---|---|---|---|---|---|
| 48 | F | Cl | HO | $CH_3S(O)$ | H | 4 | 116 - 118°C | white crystal |
| 49 | F | Cl | HO | $CH_3S(O)_2$ | H | 4 | 213 - 215°C | pale yellow crystal |
| 50 | F | Cl | $C_2H_5O$ | $CH_3S(O)_2$ | H | 4 | 113 - 115°C | pale yellow crystal |
| 51 | F | Cl | $i-C_3H_7O$ | $CH_3S(O)$ | H | 4 | 105 - 107°C | white crystal |
| 52 | F | Cl | $i-C_3H_7O$ | $CH_3S(O)_2$ | H | 4 | 130 - 133°C | pale yellow crystal |
| 53 | H | Cl | $i-C_3H_7O$ | Cl | $5-CH_3$ | 4 | 77 - 78°C | white crystal |
| 54 | H | $CH_3$ | $i-C_3H_7O$ | Cl | H | 4 | (1.5602) | yellow oil |
| 55 | H | Cl | $i-C_3H_7O$ | Cl | H | 3 | 222 - 225°C | white crystal |
| 56 | F | Cl | $CH_3OCH_2CH_2O$ | Cl | $5-CH_3$ | 4 | 62 - 63°C | white crystal |
| 57 | F | Cl | $n-C_3H_7-OCO-$<br>$CH(CH_3)-O$ | Cl | $5-CH_3$ | 4 | (1.5400) | colourless oil |
| 58 | F | Cl | $CH_3SO_2NH$ | Cl | H | 4 | 222 - 223°C | white crystal |
| 59 | F | CN | HO | Cl | H | 4 | 195 - 198°C | white crystal |
| 60 | H | Cl | $(C_2H_5)_3N^+HO^-$ | Cl | H | 4 | 85 - 88°C | white crystal |
| 61 | H | $CH_3O$ | $i-C_3H_7O$ | Cl | H | 4 | (1.5662) | yellow oil |
| 62 | H | $CH_3O$ | HO | Cl | H | 4 | 154 - 155°C | white crystal |
| 63 | H | $CH_3O$ | $CH_3O$ | Cl | H | 4 | 117 - 118°C | white crystal |

Table 1 (continued)

| | | | | | | | | |
|---|---|---|---|---|---|---|---|---|
| 64 | H | $CH_3O$ | $NH_2$ | Cl | H | 4 | 120 - 122°C | white crystal |
| 65 | $CH_3$ | H | i-$C_3H_7O$ | Cl | H | 4 | 83 - 84°C | white crystal |
| 66 | $CH_3$ | H | HO | Cl | H | 4 | 197 - 198°C | white crystal |
| 67 | H | Cl | HO | $CH_3S$ | H | 4 | 188 - 193°C | pale yellow crystal |
| 68 | H | Cl | $CH_3O$ | $CH_3S$ | H | 4 | 57 - 59°C | pale yellow crystal |
| 69 | H | Cl | $C_2H_5O$ | $CH_3S$ | H | 4 | (1.6020) | light brown oil |
| 70 | H | Cl | i-$C_3H_7O$ | $CH_3S$ | H | 4 | (1.5909) | light brown oil |
| 71 | H | Cl | n-$C_3H_7O$ | $CH_3S$ | H | 4 | (1.5949) | light brown oil |
| 72 | H | Cl | $CH_2$=$CHCH_2O$ | $CH_3S$ | H | 4 | 50 - 52°C | light brown crystal |
| 73 | H | Cl | s-$C_4H_9O$ | $CH_3S$ | H | 4 | (1.5856) | light brown oil |
| 74 | H | Cl | n-$C_5H_{11}O$ | $CH_3S$ | H | 4 | (1.5835) | light brown oil |
| 75 | F | Cl | $NH_2$ | $CH_3SO$ | H | 4 | 77 - 83°C | light brown crystal |
| 76 | F | Cl | $NH_2$ | $CH_3SO_2$ | H | 4 | 184 - 185°C | white crystal |
| 77 | H | Cl | $NH_2$ | $CH_3S$ | H | 4 | 227 - 229°C | pale yellow crystal |
| 78 | H | Cl | $CH_3NH$ | $CH_3S$ | H | 4 | 169 - 170°C | light brown crystal |

Table 1 (continued)

| | | | | | | | | |
|---|---|---|---|---|---|---|---|---|
| 79 | H | Cl | $C_2H_5NH$ | $CH_3S$ | H | 4 | 142 - 144°C | light brown crystal |
| 80 | H | Cl | $C_2H_5OCH_2CH_2-NH$ | $CH_3S$ | H | 4 | 107 - 109°C | light brown crystal |
| 81 | H | Cl | $(CH_3)_2C=N-O$ | Cl | H | 4 | 110 - 111°C | light yellow crystal |
| 82 | H | Cl | $HOCH_2-C(CH_3)_2-NH$ | Cl | H | 4 | 178 - 180°C | white crystal |
| 83 | H | Cl | $CH_3OCH_2O$ | Cl | H | 4 | (1.5838) | pale yellow oil |
| 84 | Cl | Cl | $(n-C_4H_9)_2N$ | Cl | H | 4 | 115 - 117°C | white crystal |
| 85 | H | H | $CH_3O$ | Cl | H | 4 | 76 - 77°C | white crystal |
| 86 | H | H | HO | Cl | H | 4 | 95 - 96°C | white crystal |
| 87 | H | H | $i-C_3H_7O$ | Cl | H | 4 | (1.5632) | pale yellow oil |
| 88 | H | Cl | $CH_3S$ | Cl | H | 4 | 35 - 37°C | white crystal |
| 89 | F | Cl | $i-C_3H_7O$ | $C_2H_5O$ | H | 4 | 82.5 - 83.5°C | white crystal |
| 90 | F | Cl | $i-C_3H_7O$ | $i-C_3H_7O$ | H | 4 | (1.5412) | pale yellow oil |
| 91 | F | Cl | HO | $i-C_3H_7O$ | H | 4 | 182 - 184°C | white crystal |
| 92 | Cl | Cl | HO | Cl | H | 4 | 219 - 222°C | white crystal |
| 93 | Cl | Cl | $CH_3O$ | Cl | H | 4 | 100 - 102°C | white crystal |

Table 1 (continued)

| | | | | | | | | |
|---|---|---|---|---|---|---|---|---|
| 94 | H | Cl | $CH_3O$ | Cl | H | 3 | 97 - 99°C | white crystal |
| 95 | H | Cl | $C_2H_5NH$ | Cl | H | 3 | 116 - 119°C | white crystal |
| 96 | H | Cl | $n-C_3H_7NH$ | Cl | H | 3 | 75 - 76°C | white crystal |
| 97 | H | Cl | $C_6H_5NH$ | Cl | H | 3 | 143 - 145°C | pale yellow crystal |
| 98 | H | Cl | $(CH_3)_2NH$ | Cl | H | 3 | 117 - 120°C | white crystal |
| 99 | H | Cl | $(C_2H_5)_2NH$ | Cl | H | 3 | 107 - 109°C | white crystal |
| 100 | H | Cl | $C_6H_5CH_2NH$ | Cl | H | 3 | 143 - 144°C | white crystal |
| 101 | Cl | Cl | $i-C_3H_7O$ | Cl | H | 4 | 85 - 86°C | white crystal |
| 102 | F | Cl | $NH_2$ | $CH_3S$ | H | 4 | 230°C | pale yellow crystal |
| 103 | H | $CH_3$ | HO | Cl | H | 4 | 174 - 175°C | white crystal |
| 104 | H | $CH_3$ | $CH_3O$ | Cl | H | 4 | 72 - 73°C | white crystal |
| 105 | H | $CH_3$ | $C_2H_5O$ | Cl | H | 4 | (1.5745) | pale yellow oil |
| 106 | H | Cl | $CH_2=CHCH_2NH$ | $CH_3S$ | H | 4 | 126 - 128°C | light brown crystal |
| 107 | H | Cl | $s-C_4H_9NH$ | $CH_3S$ | H | 4 | 150 - 152°C | light brown crystal |
| 108 | H | Cl | $(CH_3)_2N$ | $CH_3S$ | H | 4 | glass-like | light brown oil |

Table 1 (continued)

| | | | | | | | | |
|---|---|---|---|---|---|---|---|---|
| 109 | H | Cl | 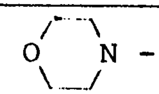 | $CH_3S$ | H | 4 | (1.5920) | light brown oil |
| 110 | F | Cl | HOOC-C $H(CH_3)NH$ | Cl | H | 4 | 187 - 191°C | white crystal |
| 111 | H | i-$C_3H_7OOC$ | i-$C_3H_7OOC$ | Cl | H | 4 | (1.5483) | pale yellow oil |
| 112 | H | HOOC | HOOC | Cl | H | 4 | 217 - 220°C | white crystal |
| 113 | H | $O_2N$ | HOOC | Cl | H | 4 | 180 - 182°C | yellow crystals |
| 114 | a mixture of 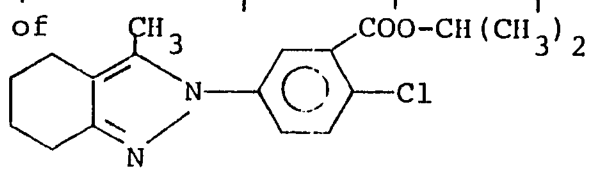 and 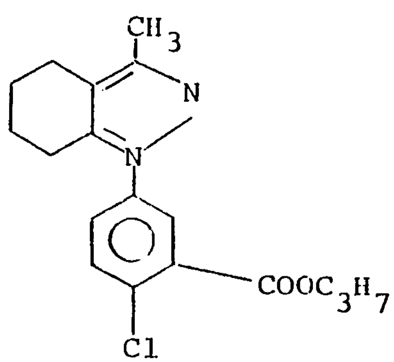 | | | | | | (1.5695) | yellow oil |

SYNTHETIC EXAMPLE 14:

Production of 1-(2-fluoro-4-chloro-5-isopropoxycarbonylphenyl)-3-methyl-5-chloropyrazole (Compound No. 119)

Into a solution of 6.0 g (0.024 mol) of 2-fluoro-4-chloro-5-isopropoxycarbonylphenylhydrazine in 100 ml of toluene, 3.0 g (0.0256 mol) of methyl acetoacetate were added, and the mixture was heated under reflux for 6 hour to carry out dehydration, and the reaction mixture was concentrated and cooled. The thus precipitated substance was filtered. To 7.9 g of thus obtained red crystals, 3.2 g (0.0264 mol) of N,N-dimethylaniline and 4.1 g (0.0267 mol) of phosphorus oxychloride were added and the mixture was heated for 3 hours at 130°C while stirring thereof. Then the reaction mixture was poured into ice water, and was extracted with chloroform. After washing the organic layer with water and drying the layer over anhydrous sodium sulfate, the dried organic layer was concentrated to obtain red substance. By purifying the red substance with a silicagel column, 7.6 g of the object compound were obtained as white crystals having a melting point of 68 to 71°C in a yield of 94 %. The thus obtained compound gave the following data of elementary analysis:

| | | C(%) | H(%) | N(%) |
|---|---|---|---|---|
| Found | : | 50.80 | 3.90 | 8.49 |
| Calcd. as $C_{14}H_{13}Cl_2FN_2O_2$ | : | 50.77 | 3.96 | 8.46 |

SYNTHETIC EXAMPLE 15:

Production of 1-(4-chloro-3-methoxycarbonylphenyl)-3,4-dimethyl-5-chloro-pyrazole (Compound No. 121)

In 100 ml of xylene, 15 g (0.0748 mol) of 4-chloro-3-methoxycarbonylphenylhydrazine and 11.8 g (0.090 mol) of ethyl α-methylacetoacetate were heated for 5 hours under reflux, and after concentrating the reaction mixture, the concentrated reaction mixture was cooled to room temperature, and the thus precipitated crystals of 1-(4-chloro-3-methoxycarbonylphenyl)-2H-4,5-dimethyl-5-pyrazolone were collected by filtration. To the thus obtained 12.1 g of the crystals corresponding to 0.0470 mol of the compound, 5.7 g (0.0470 mol) of N,N-dimethylaniline and 7.2 g (0.0470 mol) of phosphorus oxychloride were added, and the mixture was heated for 3 hours at 130°C under stirring. Then, the reaction mixture was poured into ice water and was extracted with chloroform. After concentrating the extract, the oily concentrate was purified by a silicagel column to obtain 11.6 g of the object compound as yellow crystals having a melting point of 86 to 90°C in a yield of 51.8 %. The thus obtained compound gave the following data of elementary analysis:

| | C(%) | H(%) | N(%) |
|---|---|---|---|
| Found : | 51.98 | 4.20 | 9.42 |
| Calcd. as $C_{13}H_{12}Cl_2N_2O_2$ : | 52.19 | 4.04 | 9.36 |

SYNTHETIC EXAMPLE 16:

Production of 1-(4-chloro-3-carboxyphenyl)-3,4-dimethyl-5-chloropyrazole (Compound No. 120)

Into a solution of 5 g (0.0167 mol) of 1-(4-chloro-3-methoxycarbonylphenyl)-3,4-dimethyl-5-chloropyrazole in 20 ml of ethanol, 7 g (0.035 mol) of an aqueous 20 % solution of sodium hydroxide were added and the mixture was heated for 2 hours under reflux while stirring the mixture.

Then the reaction mixture was poured into ice water, and after adjusting the mixture to weakly acidic, the thus precipitated crystals were filtered and dried. The dried amount of the white crystals were 3.5 g corresponding to a yield of 73.5 %. The crystals have a melting point of 225 to 228°C and gave the following data of elementary analysis:

| | C(%) | H(%) | N(%) |
|---|---|---|---|
| Found : | 50.82 | 3.50 | 9.91 |
| Calcd. as $C_{12}H_{10}Cl_2N_2O_2$ : | 50.55 | 3.54 | 9.82 |

SYNTHETIC EXAMPLE 17:

Production of 1-(2-fluoro-4-chloro-5-isopropoxycarbonylphenyl)-3,4-dimethyl-5-methoxypyrazole (Compound No. 127)

Into a solution of 8.2 g (0.0251 mol) of 1-(2-fluoro-4-chloro-5-isopropoxycarbonylphenyl)-2H-3,5-dimethyl-5-pyrazolone in a mixture of 50 ml of isopropyl alcohol and 50 ml of tetrahydrofuran, 1.2 g (0.0275 mol) of 55 % sodium hydride were added at 10°C, and after dissolving sodium hydride, 3.3 g

(0.0262 mol) of dimethyl sulfate were added to the mixture and the reaction mixture was stirred for 3 hours at 70°C. The reaction mixture was poured into ice water and was extracted with ethyl acetate. After washing the extract with water, the extract was dried over anhydrous sodium sulfate and concentrated an oil, which was purified by a silica gel column to obtain 1.1 g of the object compound as a pale yellow oil showing [$n_D^{25}$] of 1.5390 in a yield of 13.2 %. The thus obtained oily compound gave the following data of elementary analysis:

| | | C(%) | H(%) | N(%) |
|---|---|---|---|---|
| Found | : | 56.48 | 5.41 | 8.39 |
| Calcd. as $C_{16}H_{18}ClFN_2O_3$ | : | 56.39 | 5.32 | 8.22 |

SYNTHETIC EXAMPLE 18:

Production of 1-(2-fluoro-4-chloro-5-isopropoxycarbonylphenyl)-3-methyl-4,5-dichloropyrazole (Compound No. 132)

Into a solution of 3.0 g (0.0864 mol) of 1-(2-fluoro-4-chloro-5-isopropoxycarbonylphenyl)-3-methyl-5-chloropyrazole (Compound No. 119) in a mixture of 20 ml of chloroform and 50 ml of acetic acid, 0.8 g (0.0098 mol) of sodium acetate was added, and after cooling the mixture to 10°C, 0.92 g (0.0130 mol) of gaseous chlorine was blown into the mixture. After stirring the reaction mixture at 40°C for 30 min, the reaction mixture was poured into ice water and was extracted with chloroform. After washing the extract with aqueous solution of sodium hydrogen carbonate and with water, the washed extract was dried over

anhydrous sodium sulfate and condensed to obtain 3.4 g of the object compound as pale green substance having a melting point of 71 to 78°C in a yield of 100 %.

The thus obtained product showed the following data of elementary analysis:

| | C(%) | H(%) | N(%) |
|---|---|---|---|
| Found : | 46.02 | 3.43 | 7.60 |
| Calcd. as $C_{14}H_{12}Cl_3FN_2O_2$: | 45.99 | 3.31 | 7.66 |

SYNTHETIC EXAMPLE 19:

Production of 1-(2-fluoro-4-chloro-5-isopropoxycarbonylphenyl)-3-methyl-4-bromo-5-chloropyrazole (Compound No. 136)

Into a solution of 3 g (0.00864 mol) of 1-(2-fluoro-4-chloro-5-isopropoxycarbonylphenyl)-3-methyl-5-chloropyrazole (Compound No. 119) in a mixture of 20 ml of chloroform and 50 ml of acetic acid, 0.8 g (0.0098 mol) of sodium acetate was added, and after cooling the mixture to 0°C, 1.5 g of bromine (0.0094 mol) were added to the cooled mixture. After stirring the mixture for 30 min at 30°C, the reaction mixture was poured into ice water and was extracted with chloroform. After washing the organic layer with an aqueous solution of sodium hydrogen carbonate and then with water, the organic layer was dried over anhydrous sodium sulfate and concentrated obtain 3.3 g of the object compound as a pale yellow substance having a melting point of 71 to 75°C in a yield of 93 %. The thus obtained compound gave the following data of elementary analysis:

| | C(%) | H(%) | N(%) |
|---|---|---|---|
| Found : | 41.12 | 3.01 | 6.72 |
| Calcd. as $C_{14}H_{12}BrCl_2FN_2O_2$: | 41.00 | 2.95 | 6.83 |

SYNTHETIC EXAMPLE 20:

Production of 1-(2-fluoro-4-chloro-5-hydroxycarbonylphenyl)-3-methyl-4-iodo-5-chloropyrazole (Compound No. 140)

Into a solution of 1.4 g (0.00423 mol) of 1-(2-fluoro-4-chloro-5-isopropoxycarbonylphenyl)-3-methyl-5-chloropyrazole (Compound No. 119) in 10 ml of benzene, 1 g (0.00462 mol) of mercuric oxide and 1.2 g (0.00473 mol) of iodine were added, and the mixture was heated for 56 hours at 80°C while stirring. After cooling the reaction mixture to room temperature, the reaction mixture was filtered, and an aqueous solution of sodium hydrogen sulfite was added to the filtrate. The mixture was extracted with ethyl acetate, and the extract was washed with water, dried over anhydrous sodium sulfate and concentrated to obtain 1.3 g of an oil. After dissolving the oil in 10 ml of ethanol, 2 g of 20 % aqueous solution of sodium hydroxide were added to the solution, and after stirring the mixture for one hour at 50°C and cooling thereof, it was made acidic by the addition of dilute hydrochloric acid and then extracted with ethyl acetate. The extract was dried over anhydrous sodium sulfate and concentrated to obtain 0.8 g of pale yellow crystals having a melting point of 229 to 230°C in a total yield of 45.7 %. The thus obtained object compound

showed the following data of elementary analysis:

| | C(%) | H(%) | N(%) |
|---|---|---|---|
| Found : | 32.02 | 1.32 | 6.87 |
| Calcd. as $C_{11}H_6Cl_2FIN_2O_2$ : | 31.84 | 1.46 | 6.75 |

SYNTHETIC EXAMPLE 21:

Production of 1-(4-chloro-3-methoxycarbonylphenyl)-3-methyl-4-ethylthio-5-chloropyrazole (Compound No. 144)

Into 50 ml of toluene, 6.4 g (0.0319 mol) of 4-chloro-3-methoxycarbonylphenylhydrazine and 6 g (0.0315 mol) of ethyl α-ethylthioacetoacetate were dissolved, and after stirring the solution for 4 hours under reflux by heating to cause dehydration, the reaction mixture was cooled. Red solid substance appeared in the reaction mixture. After adding hexane to the reaction mixture, it was filtered to obtain 6.4 g of crystals. After adding 2.6 g of N,N-dimethylaniline and 3.3 g of phosphorus oxychloride to the crystals, stirring the mixture for 3 hours at 130°C and pouring the reaction mixture into ice water and was extracted with chloroform. By drying and concentrating the extract, the thus obtained oil was purified by a silicagel column to obtain 2.0 g of a yellow oil showing [$n_D^{25}$] of 1.5920 in a yield of 17.9 %. The thus obtained object compound gave the following data of elementary analysis:

| | C(%) | H(%) | N(%) |
|---|---|---|---|
| Found : | 47.61 | 3.91 | 8.01 |
| Calcd. as $C_{14}H_{14}Cl_2N_2O_2S$ : | 47.47 | 3.98 | 7.91 |

SYNTHETIC EXAMPLE 22:

Production of 1-(4-chloro-3-methoxycarbonylphenyl)-3-methyl-4-ethylsulfonyl-5-chloropyrazole (Compound No. 145)

After dissolving 1.1 g (0.0311 mol) of 1-(4-chloro-3-methoxycarbonylphenyl)-3-methyl-4-ethylthio-5-chloropyrazole (Compound No.144) in 10 ml of acetic acid, 0.7 g of aqueous 35 % solution of hydrogen peroxide was added to the solution, and the mixture was stirred for 5 hours at 0°C. The reaction mixture was poured into ice water and was extracted with ethyl acetate. The extract was dried and concentrated under a reduced pressure to obtain 0.7 g of the object compound as white crystals having a melting point of 57 to 59°C in a yield of 59.6 %. The thus obtained compound gave the following data of elementary analysis:

| | C(%) | H(%) | N(%) |
|---|---|---|---|
| Found : | 44.67 | 3.70 | 7.50 |
| Calcd. as $C_{14}H_{14}Cl_2N_2O_4S$ : | 44.57 | 3.74 | 7.42 |

Following the above-mentioned process, the present compounds shown in Table 2 and represented by the following formula were obtained.

Table 2

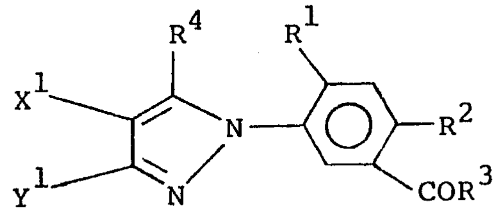

Note: * Refractive index [$n_D^{25}$]

| Compound number | Substituent $R^1$ | $R^2$ | $R^3$ | $R^4$ | $X^1$ | $Y^1$ | Melting point (or Refractive index)* | Appearance |
|---|---|---|---|---|---|---|---|---|
| 115 | H | Cl | HO | Cl | H | $CH_3$ | 215 - 217°C | white crystal |
| 116 | H | Cl | $CH_3O$ | Cl | H | $CH_3$ | 113 - 115°C | white crystal |
| 117 | H | Cl | i-$C_3H_7O$ | Cl | H | $CH_3$ | (1.5581) | light brown oil |
| 118 | F | Cl | HO | Cl | H | $CH_3$ | 205 - 207°C | white crystal |
| 119 | F | Cl | i-$C_3H_7O$ | Cl | H | $CH_3$ | 68 - 70°C | white crystal |
| 120 | H | Cl | HO | Cl | $CH_3$ | $CH_3$ | 225 - 228°C | white crystal |
| 121 | H | Cl | $CH_3O$ | Cl | $CH_3$ | $CH_3$ | 86 - 90°C | yellow crystal |
| 122 | H | Cl | i-$C_3H_7O$ | Cl | $CH_3$ | $CH_3$ | (1.5580) | yellow oil |
| 123 | F | Cl | HO | Cl | $CH_3$ | $CH_3$ | 195 - 197°C | white crystal |
| 124 | F | Cl | $CH_3O$ | Cl | $CH_3$ | $CH_3$ | 95 - 96°C | pale yellow crystal |

Table 2 (continued)

| | | | | | | | | |
|---|---|---|---|---|---|---|---|---|
| 125 | F | Cl | i-$C_3H_7O$ | Cl | $CH_3$ | $CH_3$ | 79 - 80°C | white crystal |
| 126 | Cl | Cl | HO | Cl | $CH_3$ | $CH_3$ | 183 - 189°C | pale yellow crystal |
| 127 | F | Cl | i-$C_3H_7O$ | $CH_3O$ | $CH_3$ | $CH_3$ | (1.5390) | pale yellow oil |
| 128 | H | Cl | HO | Cl | Cl | $CH_3$ | 230°C < | pale red crystal |
| 129 | H | Cl | $CH_3O$ | Cl | Cl | $CH_3$ | 106 - 107°C | pale yellow crystal |
| 130 | H | Cl | i-$C_3H_7O$ | Cl | Cl | $CH_3$ | (1.5612) | pale red oil |
| 131 | F | Cl | HO | Cl | Cl | $CH_3$ | 217 - 219°C | pale yellow crystal |
| 132 | F | Cl | i-$C_3H_7O$ | Cl | Cl | $CH_3$ | 71 - 78°C | pale green crystal |
| 133 | H | Cl | HO | Cl | Br | $CH_3$ | 227 - 235°C | white crystal |
| 134 | H | Cl | i-$C_3H_7O$ | Cl | Br | $CH_3$ | (1.5750) | pale red oil |
| 135 | F | Cl | HO | Cl | Br | $CH_3$ | 204 - 211°C | pale yellow crystal |
| 136 | F | Cl | i-$C_3H_7O$ | Cl | Br | $CH_3$ | 71 - 75°C | pale yellow crystal |
| 137 | Cl | Cl | HO | Cl | Br | $CH_3$ | 190 - 201°C | white crystal |
| 138 | Cl | Cl | $CH_3O$ | Cl | Br | $CH_3$ | 117 - 119°C | white crystal |

Table 2 (continued)

| | | | | | | | | |
|---|---|---|---|---|---|---|---|---|
| 139 | Cl | Cl | i-$C_3H_7O$ | Cl | Br | $CH_3$ | 99 - 104°C | pale yellow crystal |
| 140 | F | Cl | HO | Cl | I | $CH_3$ | 229 - 230°C | pale yellow crystal |
| 141 | H | Cl | HO | Cl | n-$C_3H_7$ | $CH_3$ | 114 - 115°C | white crystal |
| 142 | H | Cl | $CH_3O$ | Cl | n-$C_3H_7$ | $CH_3$ | (1.5696) | pale yellow oil |
| 143 | H | Cl | i-$C_3H_7O$ | Cl | n-$C_3H_7$ | $CH_3$ | (1.5502) | pale brown oil |
| 144 | H | Cl | $CH_3O$ | Cl | $C_2H_5S$ | $CH_3$ | (1.5920) | yellow oil |
| 145 | H | Cl | $CH_3O$ | Cl | $C_2H_5S(O)_2$ | $CH_3$ | 57 - 59°C | white crystal |
| 146 | H | Cl | HO | Cl | t-$C_4H_9S$ | $CH_3$ | 63 - 73°C | yellow crystal |
| 147 | F | Cl | $NH_2$ | Cl | $CH_3$ | $CH_3$ | 184 - 189°C | white crystal |
| 148 | F | Cl | $CH_3SO_2NH$ | Cl | $CH_3$ | $CH_3$ | 89 - 90°C | pale yellow crystal |
| 149 | F | Cl | HOOC-CH($CH_3$)-NH | Cl | Br | $CH_3$ | 176 - 179°C | pale yellow crystal |
| 150 | H | Cl | i-$C_3H_7O$ | Cl | i-$C_3H_7$ | $CH_3$ | (1.5518) | pale yellow oil |

Table 2 (continued)

| | | | | | | | | |
|---|---|---|---|---|---|---|---|---|
| 151 | H | Cl | HO | Cl | i-$C_3H_7$ | $CH_3$ | (1.5655) | pale yellow oil |
| 152 | H | Cl | $CH_3O$ | Cl | i-$C_3H_7$ | $CH_3$ | (1.5679) | pale yellow oil |
| 153 | H | Cl | $NH_2$ | Cl | i-$C_3H_7$ | $CH_3$ | 159 - 160°C | white crystal |
| 154 | F | Cl | HO | Cl | i-$C_3H_7$ | $CH_3$ | 143 - 145°C | pale yellow crystal |
| 155 | H | Cl | $Na^+O^-$ | Cl | $CH_3$ | $CH_3$ | 290°C < | white crystal |
| 156 | H | Cl | $(Ca^{2+})_{1/2}O^-$ | Cl | $CH_3$ | $CH_3$ | 290°C < | white crystal |
| 157 | H | Cl | $(C_2H_5NH_3)^+O^-$ | Cl | $CH_3$ | $CH_3$ | 110 - 112°C | pale yellow crystal |
| 158 | H | Cl | $NH_4^+O^-$ | Cl | $CH_3$ | $CH_3$ | 208 - 210°C | white crystal |

Of the present compounds, the preferable ones are those represented by the formula (XIX):

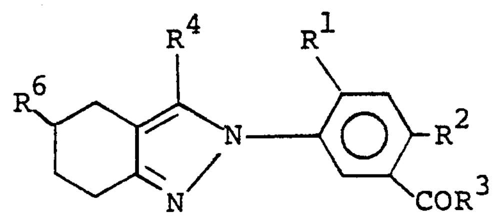

(XIX)

wherein $R^1$ is a hydrogen atom, a chlorine atom or a fluorine atom; $R^2$ is a hydrogen atom, a chlorine atom or a bromine atom, a cyano group or a methyl group; $R^3$ is a hydroxy group, an amino group, a ($C_1$ to $C_3$)-alkoxy group or a ($C_1$ to $C_3$)-alkylamino group; $R^4$ is a chlorine atom, a methoxy group or a $CH_3S(O)_m$ group wherein m is 0, 1 or 2 and $R^6$ is a hydrogen atom or a methyl group, and those represented by the formula (XX):

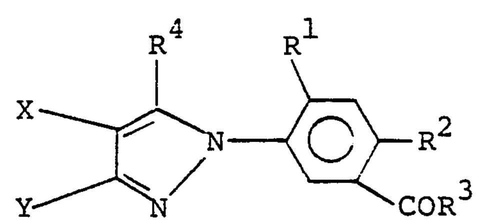

(XX)

wherein $R^1$ is a hydrogen atom, a chlorine atom or a fluorine atom; $R^2$ is a chlorine atom, $R^3$ is a hydroxy group or a ($C_1$ to $C_3$)-alkoxy group; $R^4$ is a chlorine atom or a methoxy group; X is a bromine atom or a methyl group and Y is a methyl group, and the more preferable ones are those represented by the

formula (XIX) wherein $R^1$ is a hydrogen atom or a fluorine atom; $R^2$ is a chlorine atom, a bromine atom, a methyl group or a cyano group; $R^3$ is a hydroxy group, an amino group, a methoxy group, an isopropoxy group or a methylsulfonylamino group; $R^4$ is a chlorine atom, a methoxy group or a methylthio group and $R^6$ is a hydrogen atom or a methyl group, and those represented by the formula (XX) wherein $R^1$ is a hydrogen atom or a fluorine atom; $R^2$ is a chlorine atom; $R^3$ is a hydroxy group or a methoxy group; $R^4$ is a chlorine atom and both X and Y are respectively a methyl group, or X is a bromine atom and Y is a methyl group and in addition, the most preferable compounds are the compound represented by the formula:

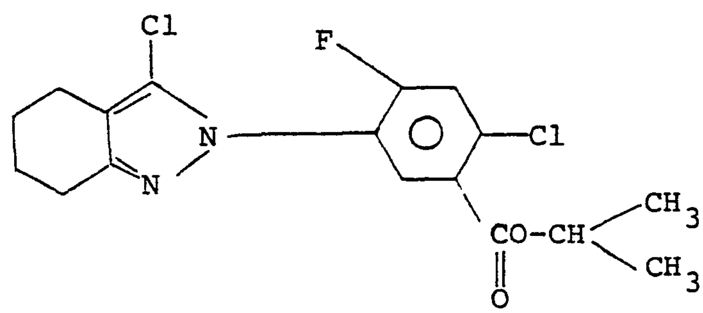

,

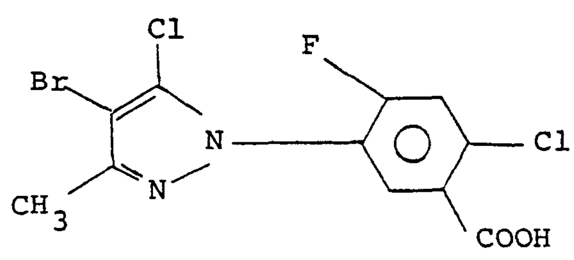

,

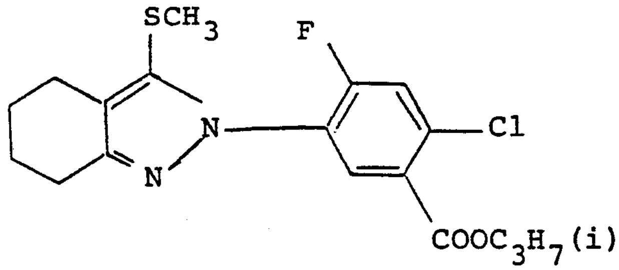

,

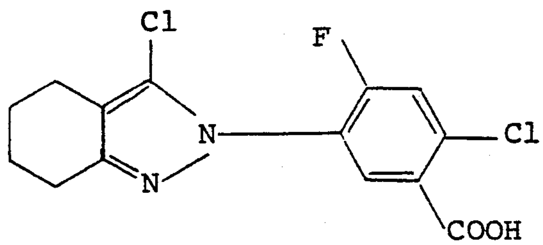

,

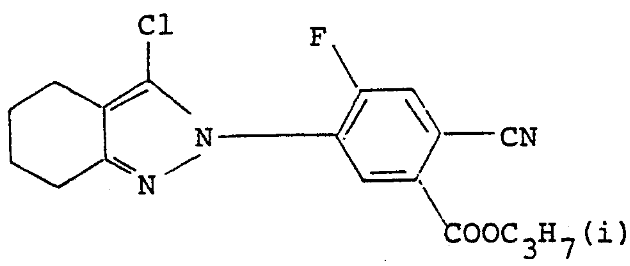

,

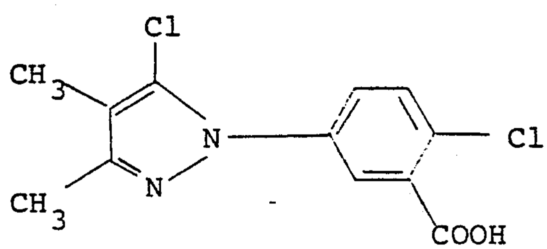

,

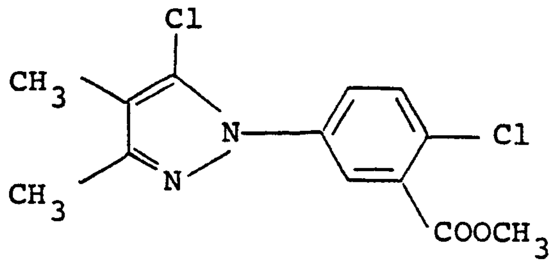

,

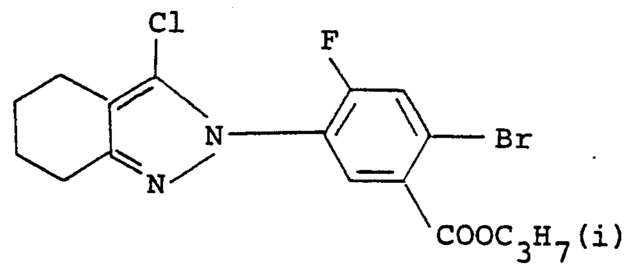

,

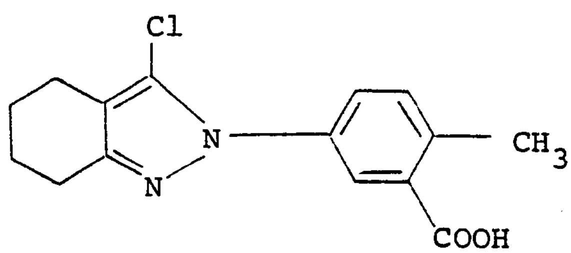

,

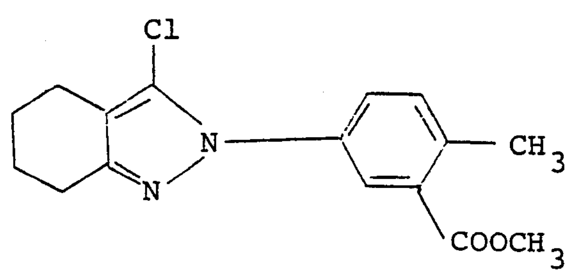

,

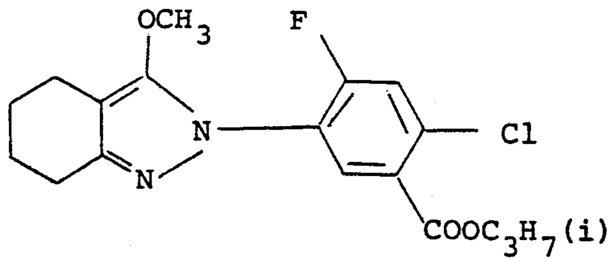

,

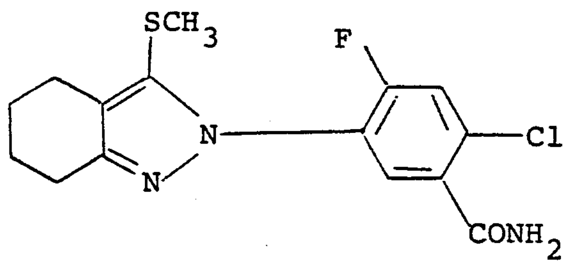

.

In the case where the present compound is used for a herbicidal composition, according to the purpose of promoting or stabilizing the effect thereof, it may be used in the form of formulation such as dust, micro granule, granule, emulsion, wettable powder and flowable suspension concentrate by mixing with adjuvants in a conventional method.

These various formulations may be used on actual application as it is or after being diluted to a desirable concentration by water. As the adjuvants carriers (diluents) and other adjuvants, for instance, extenders, emulsifiers, wetting agents, dispersing agents, disintegrators and fixing agents may be mentioned. Namely as a liquid carrier, aromatic hydrocarbons such as toluene, xylene and methylnaphthalene, aliphatic hydrocarbons such as cyclohexane, ligroine and kerosene, alcohols such as methanol, butanol and glycols, ketones such as acetone, amides such as dimethylformamide, sulfoxides such as dimethylsulfoxide, animal- and vegetable oils, fatty acids and esters of fatty acid may be mentioned. As a solid carrier, clay, kaolin, talc, diatomaceous earth, calcium carbonate, montmorillonite, bentonite, feldspar, quartz, alumina and sawdust may be mentioned.

As the emulsifier or the dispersing agent, ordinarily a surfactant is used, for instance, an anionic surfactant such as sodium higher alkylsulfate, stearyl-trimethylammonium

chloride, polyoxyethylenealkylphenyl ether and laurylbetain, a nonionic surfactant and an amphoteric surfactant may be mentioned. As the extender, for instance, polyoxyethylene nonylphenyl ether and polyoxyethylene lauryl ether may be mentioned, and as the wetting agent, for instance, dialkyl sulfosuccinate and polyoxyethylene nonylphenyl ether may be mentioned. As the fixing agent, carboxymethylcellulose and polyvinyl alcohol may be mentioned, and as the disintegrator, sodium ligninsulfonate and sodium laurylsulfate may be mentioned. Every herbicidal composition mentioned above can be used not only alone but in combination with fungicides, insecticides, acaricides, nematicides, plant growth regulators or soil improving agents. In addition, the herbicidal composition according to the present invention can be used after mixing with any fertilizer(s) and other herbicide(s).

The content of the present compound in the herbicidal composition according to the present invention depends on the type and form of formulation, the method of application and other conditions, and although there are cases where only the present compound is applied, ordinarily, the content of the present compound is in a range of from 0.5 to 95 % by weight, preferably from 2 to 50 % by weight.

In the case where weed-control is carried out, the amount of application of the herbicidal composition according to the present invention depends on the present compound used in the herbicidal composition and the fields to which the

herbicidal composition is applied, however, in general, 0.1 to 100 g, preferably 1 to 10 g of one of the present compound is applied per one are (100 $m^2$) of the field.

The herbicidal composition according to the present invention is explained more in detail.

FORMULATION EXAMPLE 1: (Emulsion)

Into 35 parts by weight of a mixture (1 : 1) of xylene and methylnaphthalene, 50 parts by weight of the present compound No. 9 were dissolved, and the thus formed solution was mixed with a mixture (8 : 2) of polyoxyethylene alkylphenyl ether and calcium alkylbenzenesulfonate to obtain an emulsion. In application, the thus formulated composition is diluted with water, thereby obtaining an aqueous emulsion containing 0.01 to 1 % by weight of the present compound No. 9.

FORMULATION EXAMPLE 2: (Dust)

To 95 parts by weight of clay, 5 parts by weight of the present compound No. 15 were added, and by blending and pulverizing the mixture, a dust was obtained. It is directly applied onto the ground where the weeds are grown, or are expected to grow.

FORMULATION EXAMPLE 3: (Wettable powder)

A mixture of 50 parts by weight of the present compound No. 25, 10 parts by weight of diatomaceous earth and 32 parts

by weight of kaolin was blended with 8 parts by weight of a mixture (1 : 1) of sodium laurylsulfate and sodium 2,2'-dinaphthylmethanesulfonate, and by pulverizing the mixture, a wettable powder was obtained.

In application of the thus obtained composition, it is diluted with water to be an aqueous suspension containing 0.01 to 2 % by weight of the present compound No. 25.

FORMULATION EXAMPLE 4: (Granule)

5 parts of a fine dust of Compound No. 26 are extended for coating on 94.5 parts of grains (16 to 32 mesh) of silica to obtain a granule, by using a methanol solution of 0.5 parts of polyvinyl polyacetate as a binding agent in a proper mixer. The granule is a scattered directly in up-land field and paddy field.

FORMULATION EXAMPLE 5: (Flowable suspension concentrate)

Into an aqueous mixture of 3 parts by weight of sodium ligninsulfonate, 0.5 part by weight of a silicone defoamer, 5 parts by weight of Acrysol® FL 104F (made by Kao Soap Co., Ltd.) and 51 parts by weight of water, the minute particles of 40 parts by weight of the present compound No. 39 were completely dispersed while stirring the mixture in a homogenizer, and 0.5 part by weight of paraformaldehyde was mixed with the thus prepared mixture to obtain a flowable suspension concentrate.

Before application, it was diluted with water so that the concentration of the compound No. 39 in the thus diluted liquid becomes to 0.01 to 2 % by weight.

The excellent herbicidal activity of a compound of the present invention will be illustrated in the following test examples.

Each test was carried out on 2-replication system and the test results are given in the average value.

TEST EXAMPLE 1 : Pre-emergence treatment in flooded condition

A fixed amount of paddy field soil was filled in each Wagner pot sized 1/5,000 are to provide a condition similar to a paddy field and there was sown a fixed amount of seeds of barnyard grass, monochoria, toothcup, false pimpernal, water wort, blurush and umbrella plant.

In addition tubers of arrowhead were buried 1 cm under the surface of soil at the rate of 3 pieces per pot and the pot was flooded with water 3 cm deep. Then the pot was applied with a diluted solution of the compound of the present invention at a rate of 0.4 to 25 g of the compound of the present invention per are.

After three days 3 pieces of rice seedlings (variety : Nihonbare) in 2.5-leaf stage were transplanted from a nursery to each pot. Thirty days after the treatment the herbicidal activity and the phytotoxicity against paddy rice were observed.

0138527

The test results were classified on the following basis as shown in Table 3.

Herbicidal activity index:

| | |
|---|---|
| 5 | Complete weeding |
| 4 | up to 80% " |
| 3 | " 60% " |
| 2 | " 40% " |
| 1 | " 20% " |
| 0 | no effect |

Phytotoxicity index:

| | |
|---|---|
| - | no damage |
| + | slight damage |
| ++ | some " |
| +++ | moderate " |
| ++++ | heavy " |
| x | complete death |

0138527

Table 3

Pre-emergence treatment under flooded condition

| Compound No. | Dosage g/are | Herbicidal effect to Barnyard grass | Broad leaf 1) | Umbrella plant | Arrow-head | Blurush | Phytotoxicity to paddy rice |
|---|---|---|---|---|---|---|---|
| 1 | 1.5 | 5 | 5 | 5 | 4.5 | 4 | - |
| | 3.2 | 5 | 5 | 5 | 5 | 4.5 | - |
| | 6.3 | 5 | 5 | 5 | 5 | 5 | + |
| 2 | 1.5 | 5 | 5 | 5 | 4.5 | 4 | - |
| | 3.2 | 5 | 5 | 5 | 4.8 | 4.5 | - |
| | 6.3 | 5 | 5 | 5 | 5 | 5 | + |
| 3 | 3.2 | 5 | 5 | 5 | 4 | 4 | - |
| | 6.3 | 5 | 5 | 5 | 4.8 | 4.5 | - |
| | 12.5 | 5 | 5 | 5 | 5 | 5 | - |
| 4 | 1.5 | 5 | 5 | 5 | 4.5 | 4.5 | - |
| | 3.2 | 5 | 5 | 5 | 5 | 5 | - |
| | 6.3 | 5 | 5 | 5 | 5 | 5 | + |
| 5 | 1.5 | 5 | 5 | 5 | 4.8 | 4 | - |
| | 3.2 | 5 | 5 | 5 | 5 | 5 | - |
| | 6.3 | 5 | 5 | 5 | 5 | 5 | + |
| 6 | 3.2 | 5 | 5 | 5 | 3 | 3 | - |
| | 6.3 | 5 | 5 | 5 | 4.5 | 4 | - |
| | 12.5 | 5 | 5 | 5 | 5 | 5 | - |
| 7 | 1.5 | 5 | 5 | 5 | 5 | 5 | - |
| | 3.2 | 5 | 5 | 5 | 5 | 5 | - |
| | 6.3 | 5 | 5 | 5 | 5 | 5 | - |
| 8 | 1.5 | 5 | 5 | 5 | 5 | 5 | - |
| | 3.2 | 5 | 5 | 5 | 5 | 5 | - |
| | 6.3 | 5 | 5 | 5 | 5 | 5 | + |
| 9 | 0.8 | 5 | 5 | 5 | 5 | 5 | - |
| | 1.5 | 5 | 5 | 5 | 5 | 5 | ± |
| | 3.2 | 5 | 5 | 5 | 5 | 5 | + |

Table 3 (continued)



| | | | | | | | |
|---|---|---|---|---|---|---|---|
| | 0.8 | 5 | 5 | 5 | 5 | 5 | + |
| 10 | 1.5 | 5 | 5 | 5 | 5 | 5 | + |
| | 3.2 | 5 | 5 | 5 | 5 | 5 | ++ |
| | 0.8 | 5 | 5 | 5 | 5 | 5 | - |
| 13 | 1.5 | 5 | 5 | 5 | 5 | 5 | - |
| | 3.2 | 5 | 5 | 5 | 5 | 5 | + |
| | 0.8 | 4.8 | 5 | 5 | 5 | 5 | - |
| 14 | 1.5 | 4.8 | 5 | 5 | 5 | 5 | - |
| | 3.2 | 5 | 5 | 5 | 5 | 5 | ++ |
| | 0.4 | 3 | 5 | 5 | 5 | 3 | - |
| 15 | 0.8 | 4 | 5 | 5 | 5 | 4 | - |
| | 1.5 | 5 | 5 | 5 | 5 | 5 | + |
| | 0.8 | 5 | 5 | 5 | 5 | 5 | - |
| 20 | 1.5 | 5 | 5 | 5 | 5 | 5 | - |
| | 3.2 | 5 | 5 | 5 | 5 | 5 | ++ |
| | 0.8 | 5 | 5 | 5 | 4 | 4 | - |
| 21 | 1.5 | 5 | 5 | 5 | 5 | 5 | + |
| | 3.2 | 5 | 5 | 5 | 5 | 5 | ++ |
| | 1.5 | 5 | 5 | 5 | 4 | 4 | - |
| 22 | 3.2 | 5 | 5 | 5 | 4.8 | 4.5 | - |
| | 6.3 | 5 | 5 | 5 | 5 | 5 | + |
| | 0.8 | 5 | 5 | 5 | 4 | 4.5 | - |
| 23 | 1.5 | 5 | 5 | 5 | 5 | 5 | - |
| | 3.2 | 5 | 5 | 5 | 5 | 5 | + |
| | 0.8 | 5 | 5 | 5 | 5 | 5 | - |
| 24 | 1.5 | 5 | 5 | 5 | 5 | 5 | - |
| | 3.2 | 5 | 5 | 5 | 5 | 5 | ++ |
| | 0.4 | 5 | 5 | 5 | 5 | 5 | - |
| 25 | 0.8 | 5 | 5 | 5 | 5 | 5 | - |
| | 1.5 | 5 | 5 | 5 | 5 | 5 | + |
| | 1.5 | 5 | 5 | 5 | 5 | 5 | - |
| 26 | 3.2 | 5 | 5 | 5 | 5 | 5 | - |
| | 6.3 | 5 | 5 | 5 | 5 | 5 | - |

Table 3 (continued)



| | | | | | | | |
|---|---|---|---|---|---|---|---|
| | 0.8 | 5 | 5 | 5 | 5 | 5 | - |
| 27 | 1.5 | 5 | 5 | 5 | 5 | 5 | - |
| | 3.2 | 5 | 5 | 5 | 5 | 5 | + |
| | 3.2 | 5 | 5 | 5 | 3 | 4 | - |
| 28 | 6.3 | 5 | 5 | 5 | 5 | 5 | - |
| | 12.5 | 5 | 5 | 5 | 5 | 5 | - |
| | 0.8 | 5 | 5 | 5 | 5 | 5 | - |
| 29 | 1.5 | 5 | 5 | 5 | 5 | 5 | + |
| | 3.2 | 5 | 5 | 5 | 5 | 5 | ++ |
| | 0.8 | 5 | 5 | 5 | 5 | 5 | - |
| 30 | 1.5 | 5 | 5 | 5 | 5 | 5 | + |
| | 3.2 | 5 | 5 | 5 | 5 | 5 | ++ |
| | 0.8 | 5 | 5 | 5 | 3 | 4 | - |
| 31 | 1.5 | 5 | 5 | 5 | 5 | 4.5 | - |
| | 3.2 | 5 | 5 | 5 | 5 | 5 | + |
| | 6.3 | 5 | 5 | 5 | 4 | 5 | - |
| 32 | 12.5 | 5 | 5 | 5 | 5 | 5 | - |
| | 25 | 5 | 5 | 5 | 5 | 5 | - |
| | 6.3 | 5 | 5 | 5 | 5 | 5 | - |
| 34 | 12.5 | 5 | 5 | 5 | 5 | 5 | + |
| | 25 | 5 | 5 | 5 | 5 | 5 | ++ |
| | 3.2 | 5 | 5 | 5 | 5 | 5 | - |
| 35 | 6.3 | 5 | 5 | 5 | 5 | 5 | - |
| | 12.5 | 5 | 5 | 5 | 5 | 5 | + |
| | 0.8 | 5 | 5 | 5 | 5 | 5 | - |
| 37 | 1.5 | 5 | 5 | 5 | 5 | 5 | - |
| | 3.2 | 5 | 5 | 5 | 5 | 5 | + |
| | 3.2 | 5 | 5 | 5 | 4.5 | - | - |
| 56 | 6.3 | 5 | 5 | 5 | 5 | - | - |
| | 12.5 | 5 | 5 | 5 | 5 | - | + |
| | 3.2 | 5 | 5 | 5 | 3 | - | - |
| 57 | 6.3 | 5 | 5 | 5 | 4 | - | - |
| | 12.5 | 5 | 5 | 5 | 5 | - | - |

Table 3 (continued)



| | | | | | | | |
|---|---|---|---|---|---|---|---|
| 58 | 1.5 | 5 | 5 | 5 | 5 | - | + |
| | 3.2 | 5 | 5 | 5 | 5 | - | + |
| | 6.3 | 5 | 5 | 5 | 5 | - | ++ |
| 78 | 6.3 | 5 | 5 | 5 | 3 | - | - |
| | 12.5 | 5 | 5 | 5 | 4 | - | - |
| 79 | 6.3 | 5 | 5 | 5 | 2 | - | - |
| | 12.5 | 5 | 5 | 5 | 3 | - | - |
| 80 | 6.3 | 5 | 5 | 5 | 4 | - | - |
| | 12.5 | 5 | 5 | 5 | 5 | - | - |
| 81 | 6.3 | 5 | 5 | 5 | 5 | - | - |
| | 12.5 | 5 | 5 | 5 | 5 | - | + |
| 82 | 1.5 | 5 | 5 | 5 | 5 | - | - |
| | 3.2 | 5 | 5 | 5 | 5 | - | + |
| | 6.3 | 5 | 5 | 5 | 5 | - | ++ |
| 83 | 6.3 | 4 | 5 | 5 | 5 | - | + |
| | 12.5 | 4.5 | 5 | 5 | 5 | - | + |
| 97 | 6.3 | 4 | 5 | 5 | 2 | - | - |
| | 12.5 | 4.5 | 5 | 5 | 3 | - | - |
| 100 | 6.3 | 5 | 5 | 5 | 2 | - | - |
| | 12.5 | 5 | 5 | 5 | 3 | - | - |
| 110 | 1.5 | 5 | 5 | 5 | 5 | - | - |
| | 3.2 | 5 | 5 | 5 | 5 | - | + |
| | 6.3 | 5 | 5 | 5 | 5 | - | ++ |
| 116 | 25 | 2 | 5 | 5 | 5 | - | - |
| | 50 | 3 | 5 | 5 | 5 | - | - |
| 117 | 12.5 | 4 | 5 | 5 | 2 | - | - |
| | 25 | 5 | 5 | 5 | 3 | - | - |
| 119 | 3.2 | 4 | 5 | 5 | 2.5 | - | - |
| | 6.2 | 5 | 5 | 5 | 3 | - | - |
| | 12.5 | 5 | 5 | 5 | 4 | - | - |
| 120 | 6.2 | 2 | 5 | 5 | 2 | - | - |
| | 12.5 | 5 | 5 | 5 | 5 | - | - |
| | 25 | 5 | 5 | 5 | 5 | - | - |

Table 3 (continued)

0138527

| | | | | | | | |
|---|---|---|---|---|---|---|---|
| 121 | 6.2 | 5 | 5 | 5 | 3 | - | - |
| | 12.5 | 5 | 5 | 5 | 5 | - | - |
| | 25 | 5 | 5 | 5 | 5 | - | - |
| 122 | 12.5 | 5 | 5 | 5 | 2 | - | - |
| | 25 | 5 | 5 | 5 | 3 | - | - |
| 123 | 3.2 | 3 | 5 | 5 | 4.5 | - | - |
| | 6.2 | 4 | 5 | 5 | 5 | - | + |
| | 12.5 | 5 | 5 | 5 | 5 | - | ++ |
| 124 | 3.2 | 5 | 5 | 5 | 4.5 | - | - |
| | 6.2 | 5 | 5 | 5 | 5 | - | + |
| | 12.5 | 5 | 5 | 5 | 5 | - | ++ |
| 125 | 1.6 | 5 | 5 | 5 | 3 | - | - |
| | 3.2 | 5 | 5 | 5 | 4 | - | + |
| | 6.2 | 5 | 5 | 5 | 5 | - | ++ |
| 127 | 3.2 | 5 | 5 | 5 | 4 | - | - |
| | 6.2 | 5 | 5 | 5 | 5 | - | - |
| | 12.5 | 5 | 5 | 5 | 5 | - | + |
| 131 | 3.2 | 2 | 5 | 5 | 5 | - | - |
| | 6.2 | 4 | 5 | 5 | 5 | - | - |
| | 12.5 | 5 | 5 | 5 | 5 | - | ++ |
| 132 | 0.8 | 5 | 5 | 5 | 3 | - | - |
| | 1.6 | 5 | 5 | 5 | 4 | - | - |
| | 3.2 | 5 | 5 | 5 | 5 | - | - |
| 135 | 3.2 | 2 | 5 | 5 | 5 | - | - |
| | 6.2 | 2.5 | 5 | 5 | 5 | - | - |
| | 12.5 | 3 | 5 | 5 | 5 | - | + |
| 136 | 3.2 | 5 | 5 | 5 | 4.5 | - | - |
| | 6.2 | 5 | 5 | 5 | 5 | - | - |
| | 12.5 | 5 | 5 | 5 | 5 | - | - |
| 138 | 6.2 | 5 | 5 | 5 | 4.5 | - | - |
| | 12.5 | 5 | 5 | 5 | 5 | - | - |
| 139 | 6.2 | 4 | 5 | 5 | 2 | - | - |
| | 12.5 | 4.5 | 5 | 5 | 3 | - | - |

Table 3 (continued)



| | | | | | | | |
|---|---|---|---|---|---|---|---|
| 140 | 6.2 | 4 | 5 | 5 | 5 | - | - |
| | 12.5 | 4.5 | 5 | 5 | 5 | - | - |
| 142 | 12.5 | 4 | 5 | 5 | 4 | - | - |
| | 25 | 5 | 5 | 5 | 5 | - | - |
| 144 | 12.5 | 4 | 5 | 5 | 4 | - | - |
| | 25 | 5 | 5 | 5 | 4.5 | - | - |
| 147 | 3.2 | 5 | 5 | 5 | 5 | - | - |
| | 6.2 | 5 | 5 | 5 | 5 | - | - |
| | 12.5 | 5 | 5 | 5 | 5 | - | ++ |
| known compound 2) | 6.3 | 5 | 5 | 5 | 0 | 0 | + |
| | 12.5 | 5 | 5 | 5 | 2.5 | 1 | + |
| | 25 | 5 | 5 | 5 | 3 | 2 | ++ |
| Control compound A 3) | 12.5 | 1 | 2 | 0 | 0 | 0 | - |
| | 25 | 3 | 4 | 3 | 0 | 0 | + |

(Notes)

1) Broadleaf: Mixture of barnyard grass, toothcup, false pimpernel, waterwort

2) A compound represented by the formula:

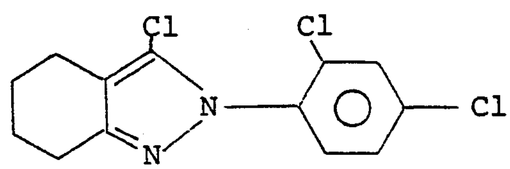

(disclosed in Japanese Patent Application Laid-Open No. 52-51365(1977)).

3) A compound represented by the formula:

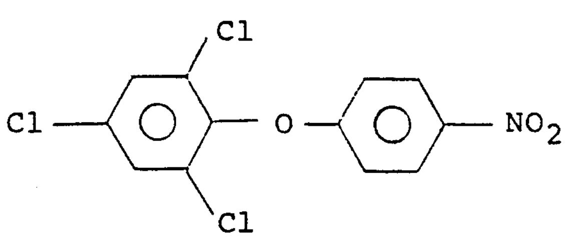

TEST EXAMPLE 2: Post-emergence treatment in flooded condition

A fixed amount of paddy field soil was filled in each Wagner pot sized 1/5,000 are to provide a condition similar to a paddy field and there was sown a fixed amount of seeds of barnyard grass, monochoria, toothcup, false pimpernel, water wort, blurush and umbrella plant.

In addition tubers of arrowhead were buried 1 cm under the surface of soil at a rate of 3 pieces per pot, three 2.5-leaf stage rice seedlings (variety : Nihonbare) were transplanted from a nursery, the pot was flooded with water 3 cm deep and then placed in a greenhouse.

When the weeds grew to reach 2 to 3-leaf stage, a diluted solution of the compound of the present invention, was applied to the flood at a rate of 1.5 - 25 g of the compound of the present invention per are.

After 30 days from the treatment with the diluted solution, the herbicidal activity was observed and obtained the results as shown in Table 4. The classification basis of the results is the same with Test Example 1.

Table 4

Post-emergence treatment in flooded condition

| Compound No. | Dosage g/are | Herbicidal effect to: Barnyard grass | Broad leaf 1) | Umbrella plant | Arrow head | Blurush | Phytotoxicity to paddy rice |
|---|---|---|---|---|---|---|---|
| 2 | 1.5 | 5 | 5 | 5 | 4.5 | 4 | - |
| | 3.2 | 5 | 5 | 5 | 5 | 5 | + |
| 3 | 12.5 | 3 | 5 | 5 | 2 | 3 | - |
| | 25 | 4 | 5 | 5 | 3 | 4 | - |
| 4 | 3.2 | 5 | 5 | 5 | 4.8 | 5 | - |
| | 6.3 | 5 | 5 | 5 | 5 | 5 | + |
| 5 | 3.2 | 5 | 5 | 5 | 4.5 | 4.5 | - |
| | 6.3 | 5 | 5 | 5 | 5 | 5 | + |
| 7 | 6.3 | 4.5 | 5 | 5 | 5 | 5 | - |
| | 12.5 | 5 | 5 | 5 | 5 | 5 | - |
| 8 | 3.2 | 5 | 5 | 5 | 4 | 5 | - |
| | 6.3 | 5 | 5 | 5 | 5 | 5 | + |
| 15 | 3.2 | 5 | 5 | 5 | 4.8 | 5 | + |
| | 6.3 | 5 | 5 | 5 | 5 | 5 | ++ |
| 21 | 6.3 | 5 | 5 | 5 | 3 | 3 | - |
| | 12.5 | 5 | 5 | 5 | 4 | 4.5 | - |
| 26 | 6.3 | 5 | 5 | 5 | 4.5 | 4.5 | - |
| | 12.5 | 5 | 5 | 5 | 5 | 5 | + |
| 28 | 12.5 | 5 | 5 | 5 | 4 | 4 | - |
| | 25 | 5 | 5 | 5 | 4.5 | 4.5 | - |
| 31 | 6.3 | 5 | 5 | 5 | 4.5 | 4.5 | - |
| | 12.5 | 5 | 5 | 5 | 5 | 5 | + |
| 32 | 12.5 | 5 | 5 | 5 | 4.8 | 5 | - |
| | 25 | 5 | 5 | 5 | 5 | 5 | - |

Table 4 (continued)



| | | | | | | | |
|---|---|---|---|---|---|---|---|
| 39 | 6.3 | 1 | 5 | 5 | 5 | 3 | - |
| | 12.5 | 2 | 5 | 5 | 5 | 5 | - |
| 56 | 6.3 | 3 | 5 | 5 | 5 | - | - |
| | 12.5 | 5 | 5 | 5 | 5 | - | - |
| 57 | 6.3 | 3 | 5 | 5 | 5 | - | - |
| | 12.5 | 4 | 5 | 5 | 5 | - | - |
| 58 | 6.3 | 3 | 5 | 5 | 5 | - | - |
| | 12.5 | 4 | 5 | 5 | 5 | - | - |
| 80 | 6.3 | 5 | 5 | 5 | 3 | - | - |
| | 12.5 | 5 | 5 | 5 | 4 | - | + |
| 81 | 6.3 | 2 | 5 | 5 | 5 | - | - |
| | 12.5 | 3 | 5 | 5 | 5 | - | - |
| 82 | 6.3 | 5 | 5 | 5 | 5 | - | - |
| | 12.5 | 5 | 5 | 5 | 5 | - | + |
| 83 | 6.3 | 2 | 5 | 5 | 5 | - | - |
| | 12.5 | 3 | 5 | 5 | 5 | - | - |
| 110 | 6.3 | 5 | 5 | 5 | 5 | - | - |
| | 12.5 | 5 | 5 | 5 | 5 | - | + |
| 119 | 6.2 | 2 | 5 | 5 | 3 | - | - |
| | 12.5 | 3 | 5 | 5 | 4 | - | - |
| 120 | 6.2 | 2 | 5 | 5 | 5 | - | - |
| | 12.5 | 2.5 | 5 | 5 | 5 | - | - |
| | 25 | 4 | 5 | 5 | 5 | - | - |
| 121 | 6.2 | 4 | 5 | 5 | 4 | - | - |
| | 12.5 | 5 | 5 | 5 | 5 | - | - |
| | 25 | 5 | 5 | 5 | 5 | - | - |
| 122 | 6.2 | 4 | 5 | 5 | 3 | - | - |
| | 12.5 | 5 | 5 | 5 | 4 | - | - |
| 123 | 12.5 | 3 | 5 | 5 | 5 | - | + |
| | 25 | 4 | 5 | 5 | 5 | - | ++ |
| 124 | 6.2 | 5 | 5 | 5 | 5 | - | - |
| | 12.5 | 5 | 5 | 5 | 5 | - | + |

Table 4 (continued)



| | | | | | | | |
|---|---|---|---|---|---|---|---|
| 125 | 3.2 | 5 | 5 | 5 | 5 | - | + |
| | 6.2 | 5 | 5 | 5 | 5 | - | ++ |
| 127 | 4.8 | 5 | 5 | 5 | 2 | - | - |
| | 12.5 | 5 | 5 | 5 | 3 | - | - |
| 131 | 12.5 | 3 | 5 | 5 | 4 | - | - |
| | 25 | 5 | 5 | 5 | 4.5 | - | - |
| 132 | 6.2 | 5 | 5 | 5 | 3 | - | - |
| | 12.5 | 5 | 5 | 5 | 4 | - | - |
| 135 | 12.5 | 2 | 5 | 5 | 3 | - | - |
| | 25 | 3 | 5 | 5 | 4 | - | - |
| 140 | 6.2 | 2 | 5 | 5 | 4.5 | - | - |
| | 12.5 | 3 | 5 | 5 | 5 | - | - |
| 147 | 6.2 | 5 | 5 | 5 | 4 | - | - |
| | 12.5 | 5 | 5 | 5 | 5 | - | - |
| known compound 4) | 6.3 | 5 | 5 | 5 | 3 | 3 | + |
| | 12.5 | 5 | 5 | 5 | 4.5 | 4 | ++ |
| known compound 2) | 12.5 | 5 | 5 | 5 | 0 | 0 | + |
| | 25 | 5 | 5 | 5 | 0 | 0 | ++ |
| Control compound A 3) | 12.5 | 0 | 1 | 1 | 0 | 0 | - |
| | 25 | 0 | 3 | 3 | 0 | 0 | - |

Notes:

4) A compound represented by the formula:

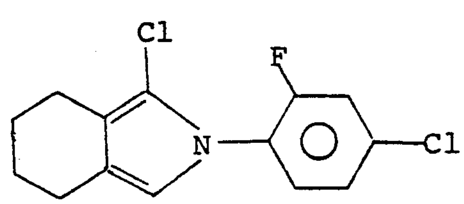

(disclosed in Japanese Patent Application Laid-Open No. 52-51365(1977))

As are seen in the results of Experimental Examples 1 and 2 shown in Tables 3 and 4, every one of the tested present compounds showed an excellent herbicidal activity to annual and perennial weed in paddy fields in pre- and post-emergence treatment and in addition, every one of the tested present compounds was quite safe to the rice seedlings when applied to the soil pre-transplanting or post-transplanting.

TEST EXAMPLE 3: Pre-emergence soil surface treatment

A fixed amount of field soil was filled in a round plastic case 8 cm across and 8 cm deep, and a fixed amount of seeds of crabgrass, foxtail, pigweed, buckweat was sown followed by covering them with soil 0.5 to 1 cm thick. Then immediately a diluted solution of the compound of the present invention was applied to treat the whole surface of soil in case at a rate of 0.8 to 50 g of the compound of the present invention per are.

After the treatment the cultivation was done in a greenhouse and the herbicidal activity was observed on the 20th day. The test was carried out on 2-replication system and each average value was sought. The judging standard of the results is the same with Test Example 1. The test results are shown in Table 5.

Table 5

Pre-emergence soil surface treatment

| Compound No. | Dosage g/are | Herbicidal effect | | | |
|---|---|---|---|---|---|
| | | foxtail | crabgrass | pigweed | buckwheat |
| 1 | 6.3 | 4.5 | 4.5 | 5 | 5 |
| | 12.5 | 5 | 5 | 5 | 5 |
| | 25 | 5 | 5 | 5 | 5 |
| 2 | 6.3 | 5 | 5 | 5 | 5 |
| | 12.5 | 5 | 5 | 5 | 5 |
| | 25 | 5 | 5 | 5 | 5 |
| 3 | 12.5 | 2 | 3 | 5 | 5 |
| | 25 | 2 | 4.5 | 5 | 5 |
| | 50 | 3 | 5 | 5 | 5 |
| 4 | 3.2 | 4.5 | 5 | 5 | 5 |
| | 6.3 | 5 | 5 | 5 | 5 |
| | 12.5 | 5 | 5 | 5 | 5 |
| 5 | 3.2 | 4.8 | 4.8 | 5 | 5 |
| | 6.3 | 5 | 5 | 5 | 5 |
| | 12.5 | 5 | 5 | 5 | 5 |
| 6 | 25 | 2 | 4 | 5 | 5 |
| | 50 | 3 | 4.5 | 5 | 5 |
| 7 | 12.5 | 4 | 4 | 5 | 5 |
| | 25 | 4.5 | 5 | 5 | 5 |
| 8 | 12.5 | 4 | 4.5 | 5 | 5 |
| | 25 | 4.5 | 5 | 5 | 5 |
| 9 | 0.8 | 4 | 5 | 5 | 5 |
| | 1.5 | 4.5 | 5 | 5 | 5 |
| | 3.2 | 5 | 5 | 5 | 5 |

Table 5 (continued)



| | | | | | |
|---|---|---|---|---|---|
| 10 | 0.8 | 5 | 5 | 5 | 5 |
| | 1.5 | 5 | 5 | 5 | 5 |
| | 3.2 | 5 | 5 | 5 | 5 |
| 13 | 25 | 2 | 4 | 5 | 5 |
| | 50 | 3 | 4.5 | 5 | 5 |
| 14 | 1.5 | 4.5 | 5 | 5 | 5 |
| | 3.2 | 5 | 5 | 5 | 5 |
| | 6.3 | 5 | 5 | 5 | 5 |
| 15 | 25 | 2 | 4.5 | 5 | 5 |
| | 50 | 4 | 4.8 | 5 | 5 |
| 20 | 6.3 | 5 | 5 | 5 | 5 |
| | 12.5 | 5 | 5 | 5 | 5 |
| 23 | 25 | 3 | 3 | 5 | 5 |
| | 50 | 4 | 4 | 5 | 5 |
| 27 | 1.5 | 4.8 | 5 | 5 | 5 |
| | 3.2 | 5 | 5 | 5 | 5 |
| | 6.3 | 5 | 5 | 5 | 5 |
| 39 | 25 | 2 | 2 | 5 | 5 |
| | 50 | 2 | 2 | 5 | 5 |
| 56 | 12.5 | 5 | 5 | 5 | 5 |
| | 25 | 5 | 5 | 5 | 5 |
| 57 | 12.5 | 2 | 2 | 5 | 5 |
| | 25 | 3 | 3 | 5 | 5 |
| 58 | 12.5 | 5 | 5 | 5 | 5 |
| | 25 | 5 | 5 | 5 | 5 |
| 78 | 12.5 | 2 | 3 | 5 | 5 |
| | 25 | 4 | 4 | 5 | 5 |
| 80 | 12.5 | 4 | 5 | 5 | 5 |
| | 25 | 5 | 5 | 5 | 5 |
| 81 | 12.5 | 1 | 1 | 5 | 5 |
| | 25 | 2 | 2 | 5 | 5 |

Table 5 (continued)



| | | | | | |
|---|---|---|---|---|---|
| 82 | 6.3 | 5 | 5 | 5 | 5 |
| | 12.5 | 5 | 5 | 5 | 5 |
| 83 | 12.5 | 1 | 1 | 5 | 5 |
| | 25 | 2 | 2 | 5 | 5 |
| 100 | 12.5 | 2 | 2 | 5 | 4 |
| | 25 | 3 | 4 | 5 | 5 |
| 110 | 12.5 | 4 | 3 | 5 | 5 |
| | 25 | 5 | 4.5 | 5 | 5 |
| 119 | 6.2 | 3 | 3 | 5 | 5 |
| | 12.5 | 4 | 4 | 5 | 5 |
| 121 | 6.2 | 2 | 3 | 5 | 5 |
| | 12.5 | 3 | 4 | 5 | 5 |
| 123 | 12.5 | 2 | 2 | 5 | 5 |
| | 25 | 3 | 3 | 5 | 5 |
| 124 | 6.2 | 3 | 4 | 5 | 5 |
| | 12.5 | 4 | 5 | 5 | 5 |
| 125 | 3.2 | 5 | 5 | 5 | 5 |
| | 6.2 | 5 | 5 | 5 | 5 |
| 127 | 6.2 | 4 | 5 | 5 | 5 |
| | 12.5 | 4.5 | 5 | 5 | 5 |
| 129 | 12.5 | 2 | 3 | 3 | 3 |
| | 25 | 2 | 3 | 5 | 5 |
| 132 | 1.6 | 5 | 5 | 5 | 5 |
| | 3.2 | 5 | 5 | 5 | 5 |
| 135 | 6.2 | 2 | 2 | 5 | 5 |
| | 12.5 | 3 | 3 | 5 | 5 |
| 136 | 3.2 | 5 | 5 | 5 | 5 |
| | 6.2 | 5 | 5 | 5 | 5 |
| 138 | 12.5 | 5 | 5 | 5 | 5 |
| | 25 | 5 | 5 | 5 | 5 |

Table 5 (continued)



| | | | | | |
|---|---|---|---|---|---|
| 144 | 12.5 | 2 | 3 | 4 | 4 |
| | 25 | 3 | 4 | 5 | 5 |
| 145 | 12.5 | 2 | 3 | 5 | 5 |
| | 25 | 3 | 3.5 | 5 | 5 |
| 148 | 3.2 | 5 | 5 | 5 | 5 |
| | 6.2 | 5 | 5 | 5 | 5 |
| 149 | 3.2 | 5 | 5 | 5 | 5 |
| | 6.2 | 5 | 5 | 5 | 5 |
| known compound 2) | 25 | 4.5 | 4.5 | 5 | 5 |
| | 50 | 4.5 | 4.5 | 5 | 5 |
| Control compound B 5) | 12.5 | 2 | 3 | 5 | 5 |
| | 25 | 4 | 4.5 | 5 | 5 |

Note:

5) A compound represented by the formula:

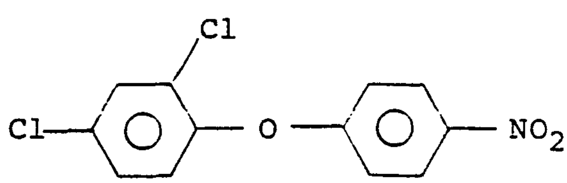

TEST EXAMPLE 4: Phytotoxicity against crops

A fixed amount of field soil was filled in a plastic vessel sized 23 cm x 4.5 cm x 12.5 cm and a fixed amount of seeds of soybean, cotton, corn, wheat, peanut and rice was sown followed by 3-cm thick covering with soil.

Then immediately a diluted solution of the compound of the present invention was sprayed on the soil surface with a small sprayer at a rate of 6.3 to 50 g of the compound of the present invention.

After the treatment the crops were grown in a greenhouse and 20 days later the degree of phytotoxicity against each crop was observed. The test was carried out on 2-replication system and each average value was sought.

The judging standard of test results is the same with Test Example 1 and the results are shown in Table 6.

Table 6



Phytotoxicity against crops (Pre-emergence treatment)

| Compound number | Dosage g/are | Effect on the following crop plant | | | | | |
|---|---|---|---|---|---|---|---|
| | | Soybean | Cotton | Peanut | Maize | Rice | Wheat |
| 1 | 25 | – | – | – | – | – | – |
| | 50 | – | – | – | – | + | + |
| 2 | 25 | – | – | – | – | – | – |
| | 50 | – | – | – | – | – | – |
| 3 | 12.5 | – | – | – | – | – | – |
| | 25 | – | – | – | – | – | – |
| 4 | 12.5 | – | – | – | – | – | – |
| | 25 | – | – | – | – | – | – |
| 5 | 12.5 | – | – | – | – | – | – |
| | 25 | – | – | – | – | – | – |
| 6 | 25 | – | – | – | – | – | – |
| | 50 | – | + | – | – | + | + |
| 7 | 25 | – | + | – | – | – | – |
| | 50 | – | ++ | – | – | – | – |
| 8 | 25 | – | + | – | – | – | – |
| | 50 | – | + | – | + | + | + |
| 9 | 6.3 | – | + | – | – | – | – |
| | 12.5 | + | ++ | + | – | – | – |
| 10 | 6.3 | + | + | – | – | – | – |
| | 12.5 | + | ++ | + | – | – | – |
| 13 | 25 | – | – | – | – | – | – |
| | 50 | + | – | – | – | – | – |
| 14 | 6.3 | + | – | – | + | – | – |
| | 12.5 | + | – | – | ++ | + | + |
| 20 | 25 | – | – | – | – | – | – |
| | 50 | + | ++ | + | + | + | + |

Table 6 (continued)



| | | | | | | | |
|---|---|---|---|---|---|---|---|
| 27 | 6.3 | + | ++ | – | + | – | – |
| | 12.5 | ++ | ++ | + | + | + | + |
| 56 | 25 | – | – | – | – | – | + |
| | 50 | + | ++ | – | + | + | ++ |
| 57 | 25 | – | – | – | – | – | – |
| | 50 | + | + | – | – | + | + |
| 58 | 25 | – | + | – | – | – | – |
| | 50 | + | ++ | + | + | – | + |
| 78 | 25 | – | – | – | – | – | – |
| | 50 | – | ++ | – | – | – | + |
| 80 | 25 | – | – | – | – | – | – |
| | 50 | – | + | – | – | + | + |
| 81 | 25 | – | – | – | – | – | – |
| | 50 | – | ++ | + | + | – | + |
| 82 | 12.5 | – | – | – | – | – | – |
| | 25 | – | + | – | – | – | + |
| 83 | 25 | – | – | – | – | – | – |
| | 50 | – | ++ | – | – | – | + |
| 100 | 25 | – | – | – | – | – | – |
| | 50 | – | – | – | – | – | – |
| 110 | 25 | – | – | – | – | – | – |
| | 50 | – | – | – | – | – | – |
| 119 | 12.5 | – | – | – | – | – | – |
| | 25 | – | + | – | + | – | + |
| 121 | 12.5 | – | – | – | – | – | – |
| | 25 | – | – | – | – | – | – |
| 123 | 25 | – | + | – | – | – | – |
| | 50 | + | ++ | + | ++ | – | – |
| 124 | 12.5 | – | – | – | – | – | – |
| | 25 | + | + | + | ++ | – | – |

Table 6 (continued)



| | | | | | | | |
|---|---|---|---|---|---|---|---|
| 125 | 6.2 | + | + | + | – | – | – |
| | 12.5 | ++ | ++ | + | + | – | – |
| 127 | 12.5 | – | – | – | – | – | – |
| | 25 | – | – | – | – | – | – |
| 129 | 25 | – | – | – | – | – | – |
| | 50 | – | – | – | – | – | – |
| 132 | 3.2 | – | – | – | – | – | – |
| | 6.2 | + | + | + | ++ | – | – |
| 135 | 12.5 | – | – | – | – | – | – |
| | 25 | – | – | – | – | – | – |
| 136 | 6.2 | – | – | – | – | – | – |
| | 12.5 | – | – | – | – | – | – |
| 138 | 25 | – | – | – | – | – | – |
| | 50 | – | – | – | – | – | – |
| 144 | 25 | – | – | – | – | – | – |
| | 50 | – | – | – | – | – | – |
| 145 | 25 | – | – | – | – | – | – |
| | 50 | ++ | ++ | + | + | – | – |
| 148 | 6.2 | – | – | – | – | – | – |
| | 12.5 | – | – | – | – | – | – |
| 149 | 6.2 | – | – | – | – | – | – |
| | 12.5 | – | – | – | – | – | – |
| known compound 2) | 25 | + | + | + | ++ | ++ | ++ |
| | 50 | ++ | ++ | ++ | +++ | +++ | ++ |
| Control compound B 5) | 25 | + | +++ | + | ++ | + | + |
| | 50 | +++ | X | ++ | X | ++ | ++ |

EXPERIMENTAL EXAMPLE 5:

Post emergence treatment

Into the soil of up-land in a plastic pot of 8 cm in diameter and 8 cm in depth, a predetermined amount of seeds of the following species of weeds in Table 7 was sown, and the pots were kept to grow the weeds.

And when the weeds are in the 3 to 4 leaf stage, a liquid which had been prepared by diluting a wettable powder formulated using the compounds of Table 7 so as to apply 12.5 to 50 g of the present compound per 100 $m^2$ of the surface of the soil in the pot, was sprayed onto the weeds in the pot. After 20 days, the herbicidal effect on the weeds was investigated, the extent of herbicidal effect being indexed according to Experimental Example 1 and shown in Table 7.

Table 7

Post emergence treatment

| Compound number | Dosage g/are | Herbicidal effect | | | |
|---|---|---|---|---|---|
| | | foxtail | crabgrass | pigweed | buckwheat |
| 1 | 12.5 | 4 | 3 | 5 | 5 |
| | 25 | 4.5 | 4 | 5 | 5 |
| 2 | 12.5 | 5 | 5 | 5 | 5 |
| | 25 | 5 | 5 | 5 | 5 |
| 4 | 12.5 | 5 | 4.5 | 5 | 5 |
| | 25 | 5 | 5 | 5 | 5 |
| 7 | 12.5 | 2 | 3 | 5 | 5 |
| | 25 | 3 | 4 | 5 | 5 |
| 9 | 12.5 | 5 | 5 | 5 | 5 |
| | 25 | 5 | 5 | 5 | 5 |
| 10 | 12.5 | 5 | 5 | 5 | 5 |
| | 25 | 5 | 5 | 5 | 5 |
| 13 | 12.5 | 2 | 2 | 5 | 5 |
| | 25 | 3 | 3 | 5 | 5 |
| 15 | 12.5 | 4.5 | 4.5 | 5 | 5 |
| | 25 | 5 | 5 | 5 | 5 |
| 18 | 12.5 | 5 | 5 | 5 | 5 |
| | 25 | 5 | 5 | 5 | 5 |

Table 7 (continued)

| | | | | | |
|---|---|---|---|---|---|
| 20 | 12.5 | 5 | 5 | 5 | 5 |
| | 25 | 5 | 5 | 5 | 5 |
| 23 | 12.5 | 2 | 2 | 5 | 5 |
| | 25 | 3 | 3 | 5 | 5 |
| 24 | 12.5 | 4 | 4 | 5 | 5 |
| | 25 | 5 | 5 | 5 | 5 |
| 25 | 12.5 | 5 | 5 | 5 | 5 |
| | 25 | 5 | 5 | 5 | 5 |
| 26 | 12.5 | 3 | - | 5 | - |
| | 25 | 4 | - | 5 | - |
| 27 | 12.5 | 5 | 5 | 5 | 5 |
| | 25 | 5 | 5 | 5 | 5 |
| 29 | 12.5 | 2 | - | 4 | - |
| | 25 | 3 | - | 4.5 | - |
| 30 | 12.5 | 2 | - | 5 | - |
| | 25 | 3 | - | 5 | - |
| 33 | 12.5 | 2 | - | 5 | - |
| | 25 | 4 | - | 5 | - |
| 34 | 6.2 | 5 | - | 5 | - |
| | 12.5 | 5 | - | 5 | - |
| 35 | 6.2 | 5 | - | 5 | - |
| | 12.5 | 5 | - | 5 | - |
| 56 | 12.5 | 3 | - | 5 | - |
| | 25 | 4 | - | 5 | - |

Table 7 (continued)

0138527

| | | | | | |
|---|---|---|---|---|---|
| 57 | 12.5 | 3 | - | 5 | - |
| | 25 | 5 | - | 5 | - |
| 58 | 12.5 | 4 | - | 5 | - |
| | 25 | 5 | - | 5 | - |
| 80 | 12.5 | 2 | - | 5 | - |
| | 25 | 3 | - | 5 | - |
| 83 | 12.5 | 5 | - | 5 | - |
| | 25 | 5 | - | 5 | - |
| 118 | 12.5 | 2 | - | 5 | - |
| | 25 | 3 | - | 5 | - |
| 120 | 12.5 | 5 | - | 5 | - |
| | 25 | 5 | - | 5 | - |
| 123 | 6.2 | 2 | - | 5 | - |
| | 12.5 | 3 | - | 3 | - |
| 124 | 6.2 | 4.5 | - | 5 | - |
| | 12.5 | 5 | - | 5 | - |
| 125 | 6.2 | 5 | - | 5 | - |
| | 12.5 | 5 | - | 5 | - |
| 127 | 12.5 | 4 | - | 5 | - |
| | 25 | 5 | - | 5 | - |

Table 7 (continued)



| | | | | | |
|---|---|---|---|---|---|
| 131 | 12.5 | 2 | - | 5 | - |
| | 25 | 3 | - | 5 | - |
| 132 | 12.5 | 3 | - | 5 | - |
| | 25 | 4 | - | 5 | - |
| 135 | 12.5 | 2 | - | 5 | - |
| | 25 | 3 | - | 5 | - |
| 136 | 12.5 | 3 | - | 5 | - |
| | 25 | 4.5 | | 5 | - |
| known compound 2) | 12.5 | 1 | 2 | 3 | 2 |
| | 25 | 2 | 3 | 4.5 | 4 |
| Control compound B 5) | 6.2 | 0 | - | - | - |
| | 12.5 | 2 | 3 | 4.5 | 4.5 |
| | 25 | 3 | 3.5 | 5 | 5 |

As are seen in the results of Experimental Examples 3 and 5, the present compound exhibits an excellent herbicidal activity on the major weeds in pre- and post-emergence treatments, crop fields and as are seen in the results of Experimental Example 4, the present compound does not harm the crop plants in the up land and accordingly, the present compound can be suitably used as a herbicide in the ordinary up-land.

CLAIMS FOR CONTRACTING STATES BE, CH, DE, FR, GB, IT, LI AND NL

1. A compound, suitable for use as a herbicide, represented by the formula (I):

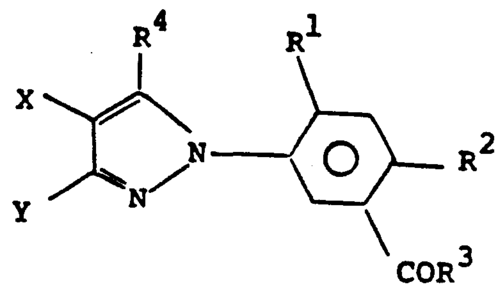

(I)

wherein $R^1$ represents hydrogen, halogen or methyl;

$R^2$ represents hydrogen, halogen, nitro, methyl, cyano, carboxy, ($C_1$ to $C_8$)-alkoxy or ($C_1$ to $C_4$)-alkoxycarbonyl;

$R^3$ represents hydroxy, amino, ($C_3$ or $C_4$)-alkenyloxy, ($C_3$ or $C_4$)-alkynyloxy, ($C_1$ to $C_4$)-alkoxy-($C_1$ to $C_4$)-alkoxy, ($C_1$ to $C_4$)-alkylthio, ($C_3$ or $C_4$)- alkenylamino, ($C_1$ to $C_4$)-alkylsulfonylamino, ($C_1$ or $C_2$)-alkoxy-($C_1$ or $C_2$)-alkylamino, $-O-N=C(CH_3)_2$, anilino, benzylamino, morpholino, ($C_1$ to $C_6$)-alkoxy unsubstituted or substituted by ($C_1$ to $C_4$)-alkoxycarbonyl, ($C_1$ to $C_4$)-mono- or di-alkylamino group in which the or each alkyl group is unsubstituted or substituted by hydroxy or carboxy, or -OM wherein M represents an alkali metal, alkaline earth metal, ammonium or ($C_1$ to $C_4$)-alkylammonium;

$R^4$ represents halogen, methyl, ($C_1$ to $C_4$)-alkoxy, $-S(O)_m-R^5$ wherein m is 0, 1 or 2 and $R^5$ represents ($C_1$ to $C_4$)-alkyl;

X represents hydrogen, halogen, ($C_1$ to $C_4$)-alkyl, ($C_1$ to $C_4$)-alkylthio or ($C_1$ to $C_4$)-alkylsulfonyl;and

Y represents ($C_1$ to $C_4$)-alkyl or X and Y taken together with the atoms to which they are attached form a cyclopentyl or cyclohexyl ring unsubstituted or substituted with one or more methyl group.

2. A compound according to claim 1, represented by the formula:

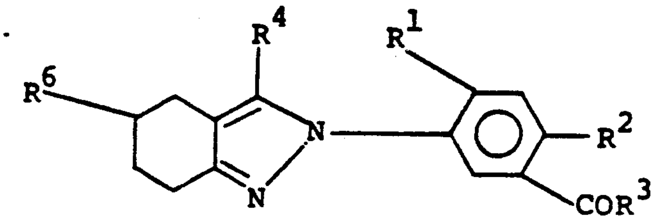

wherein $R^1$ represents hydrogen, chlorine or fluorine;

$R^2$ represents hydrogen, chlorine, bromine, cyano or methyl;

$R^3$ represents hydroxy, amino, ($C_1$ to $C_3$)-alkoxy or ($C_1$ to $C_3$)-alkylamino;

$R^4$ represents chlorine, methoxy or $-S(O)_m-CH_3$ wherein m is 0, 1 or 2; and

$R^6$ represents hydrogen or methyl.

3. A compound according to claim 2, wherein $R^1$ represents hydrogen or fluorine;

$R^2$ represents chlorine, methyl or cyano;

$R^3$ represents hydroxy, amino, methoxy, isopropoxy or methylsulfonylamino

$R^4$ represents chlorine, methoxy or methylthio; and

$R^6$ represents hydrogen or methyl.

4. A compound according to claim 1, wherein $R^1$ represents hydrogen, chlorine or fluorine;

$R^2$ represents chlorine;

$R^3$ represents hydroxy or ($C_1$ to $C_3$)-alkoxy;

$R^4$ represents chlorine or methoxy;

X represents bromine or methyl and

Y represents methyl.

5. A compound according to claim 4, wherein $R^1$ represents hydrogen or fluorine;

$R^2$ represents chlorine;

$R^3$ is hydroxy or methoxy;

$R^4$ represents chlorine, and

X and Y both represent methyl or X is bromine and Y is methyl.

6. A compound according to claim 1, represented by the formula:

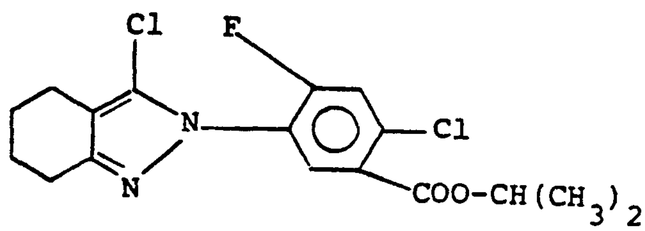

7. A compound according to claim 1, represented by the formula:

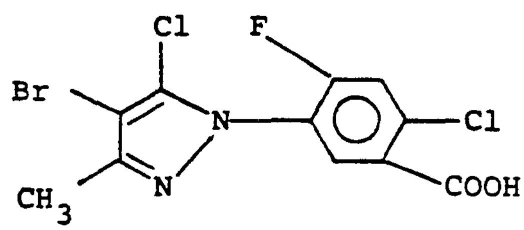

8. A process for the preparation of a compound of formula (I) as defined in claim 1 wherein $R^3$ represents hydroxy, ($C_3$ or $C_4$)-alkenyloxy, ($C_3$ or $C_4$)-alkynyloxy, ($C_1$ to $C_4$)-alkoxy-($C_1$ to $C_4$)-alkoxy, ($C_1$ to $C_4$)-alkylthio, ($C_1$ to $C_6$)-alkoxy unsubstituted or substituted by ($C_1$ to $C_4$)-alkoxycarbonyl; and $R^4$ represents halogen; which process comprises reacting a compound represented by the formula (II):

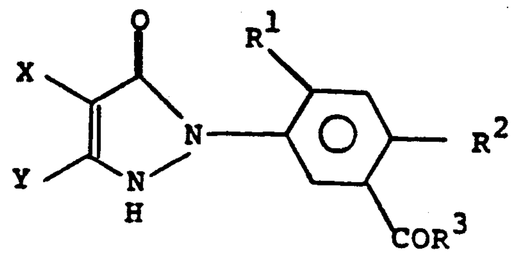

wherein $R^1$, $R^2$, X and Y are as defined in claim 1 and $R^3$ is as defined above, with a halogenating agent in an inert solvent.

9. A process for the preparation of a compound of formula (I) as defined in claim 1 wherein $R^1$, $R^2$, $R^3$, X and Y are as defined in claim 1 and $R^4$ is ($C_1$ to $C_4$)-alkoxy; which process comprises reacting a compound represented by the formula (II):

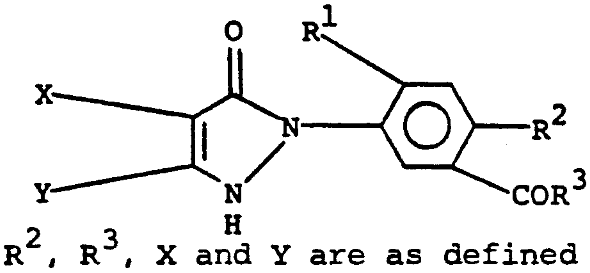

wherein $R^1$, $R^2$, $R^3$, X and Y are as defined above, with a compound represented by the formula (III):

$$(R^5O)_2SO_2$$

wherein $R^5$ is ($C_1$ to $C_4$)-alkyl, in an inert solvent and in the presence of a base.

10. A process for the preparation of a compound of formula (I) as defined in claim 1, wherein $R^1$, $R^2$, $R^3$, X and Y are as defined in claim 1 and $R^4$ is $-S(O)_m-R^5$ wherein m is 0, 1 or 2 and $R^5$ is a ($C_1$ to $C_4$)-alkyl group; which process comprises;

(1) heating a compound represented by the formula (XII):

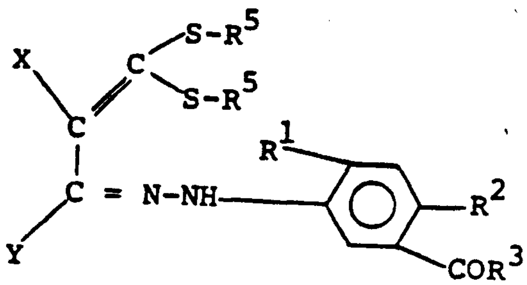

wherein $R^1$, $R^2$, $R^3$, $R^5$, X and Y are as defined above, in an inert solvent at a temperature up to the reflux temperature of the solvent, thereby obtaining a compound represented by the formula (XIII):

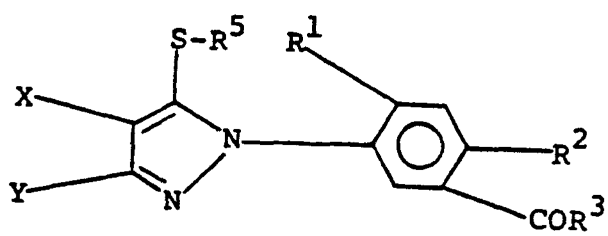

wherein $R^1$, $R^2$, $R^3$, $R^5$, X and Y are as defined above; and, if desired,

(2) reacting the compound of formula (XIII) thus-obtained with a peroxide in an inert solvent, thereby obtaining a compound represented by the formula (XIV):

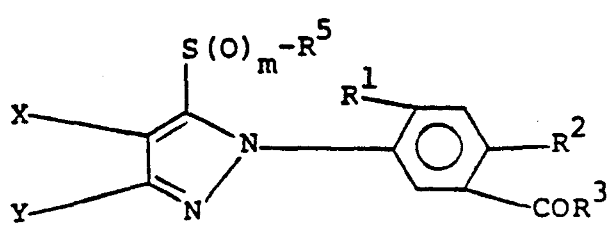

wherein $R^1$, $R^2$, $R^3$, $R^5$, X and Y are as defined above and m is 1 or 2.

11. A process for the preparation of a compound of formula (I) as defined in claim 1, which process comprises reacting a compound represented by the formula (XVI):

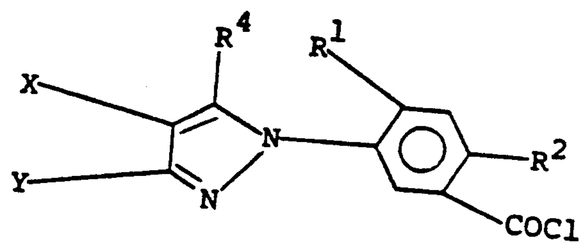

wherein $R^1$, $R^2$, $R^4$, X and Y are as defined in claim 1, with a compound represented by the formula (XVII):

$$H - R^3$$

wherein $R^3$ is as defined in claim 1, in an inert solvent.

12. A process for the preparation of a compound of formula (I) as defined in claim 1 wherein X is halogen; which process comprises reacting a compound represented by the formula (XVIII):

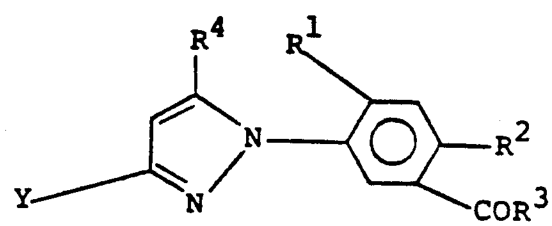

wherein $R^1$, $R^2$, $R^3$, $R^4$ and Y are as defined in claim 1, with chlorine, bromine or iodine in an inert solvent.

13. A process for the preparation of a compound of formula (I) as defined in claim 1 wherein $R^3$ is -OM wherein M is as defined in claim 1; which process comprises reacting a compound represented by the formula (XV):

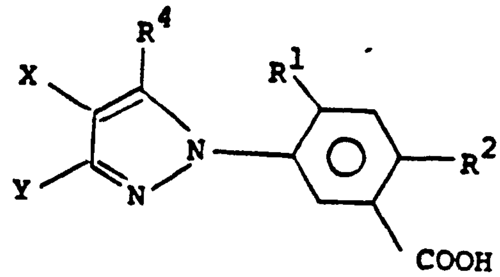

wherein $R^1$, $R^2$, $R^4$, X and Y are as defined in claim 1, with a salt of an alkali metal, an alkaline earth metal or ammonium or with a ($C_1$ to $C_4$)-alkylamine.

14. A herbicidal composition comprising a compound of formula (I) as defined in any one of claims 1 to 7 as active ingredient, together with a suitable adjuvant.

15. A method of controlling the growth of weeds at a locus, which method comprises applying to the locus a herbicidally effective amount of a compound of formula (I) as defined in any one of claims 1 to 7.

0138527

CLAIMS FOR CONTRACTING STATE AT

1. A method of controlling the growth of weeds at a locus, which method comprises applying to the locus a herbicidally effective amount of a compound represented by the formula (I):

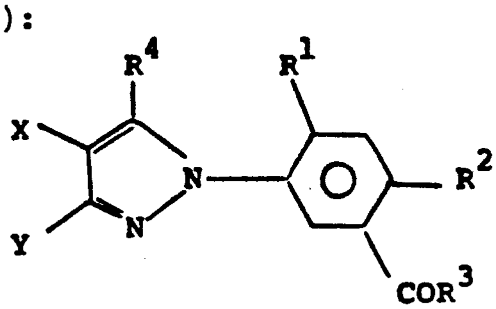

(I)

wherein $R^1$ represents hydrogen, halogen or methyl;

$R^2$ represents hydrogen, halogen, nitro, methyl, cyano, carboxy, ($C_1$ to $C_8$)-alkoxy or ($C_1$ to $C_4$)-alkoxycarbonyl;

$R^3$ represents hydroxy, amino, ($C_3$ or $C_4$)-alkenyloxy, ($C_3$ or $C_4$)-alkynyloxy, ($C_1$ to $C_4$)-alkoxy-($C_1$ to $C_4$)-alkoxy, ($C_1$ to $C_4$)-alkylthio, ($C_3$ or $C_4$)- alkenylamino, ($C_1$ to $C_4$)-alkylsulfonylamino, ($C_1$ or $C_2$)-alkoxy-($C_1$ or $C_2$)-alkylamino, $-O-N=C(CH_3)_2$, anilino, benzylamino, morpholino, ($C_1$ to $C_6$)-alkoxy unsubstituted or substituted by ($C_1$ to $C_4$)-alkoxycarbonyl, ($C_1$ to $C_4$)-mono- or di-alkylamino group in which the or each alkyl group is unsubstituted or substituted by hydroxy or carboxy, or -OM wherein M represents an alkali metal, alkaline earth metal, ammonium or ($C_1$ to $C_4$)-alkylammonium;

$R^4$ represents halogen, methyl, ($C_1$ to $C_4$)-alkoxy, $-S(O)_m-R^5$ wherein m is 0, 1 or 2 and $R^5$ represents ($C_1$ to $C_4$)-alkyl;

X represents hydrogen, halogen, ($C_1$ to $C_4$)-alkyl, ($C_1$ to $C_4$)-alkylthio or ($C_1$ to $C_4$)-alkylsulfonyl;and

Y represents ($C_1$ to $C_4$)-alkyl or X and Y taken together with the atoms to which they are attached form a cyclopentyl or cyclohexyl ring unsubstituted or substituted with one or more methyl group.

2. A method according to claim 1, wherein the compound of formula (I) is represented by the formula:

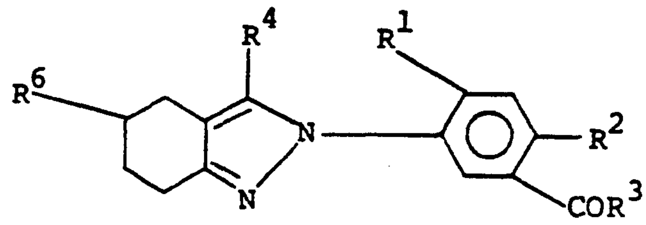

wherein $R^1$ represents hydrogen, chlorine or fluorine;
$R^2$ represents hydrogen, chlorine, bromine, cyano or methyl;
$R^3$ represents hydroxy, amino, ($C_1$ to $C_3$)-alkoxy or ($C_1$ to $C_3$)-alkylamino;
$R^4$ represents chlorine, methoxy or $-S(O)_m-CH_3$ wherein m is 0, 1 or 2; and
$R^6$ represents hydrogen or methyl.

3. A method according to claim 2,
wherein $R^1$ represents hydrogen or fluorine;
$R^2$ represents chlorine, methyl or cyano;
$R^3$ represents hydroxy, amino, methoxy, isopropoxy or methylsulfonylamino
$R^4$ represents chlorine, methoxy or methylthio; and
$R^6$ represents hydrogen or methyl.

4. A method according to claim 1,
wherein $R^1$ represents hydrogen, chlorine or fluorine;
$R^2$ represents chlorine;
$R^3$ represents hydroxy or ($C_1$ to $C_3$)-alkoxy;
$R^4$ represents chlorine or methoxy;
X represents bromine or methyl and
Y represents methyl.

5. A method according to claim 4,
wherein $R^1$ represents hydrogen or fluorine;
$R^2$ represents chlorine;
$R^3$ is hydroxy or methoxy;
$R^4$ represents chlorine, and

X and Y both represent methyl or X is bromine and Y is methyl.

6. A method according to claim 1, wherein the compound of formula (I) is represented by the formula:

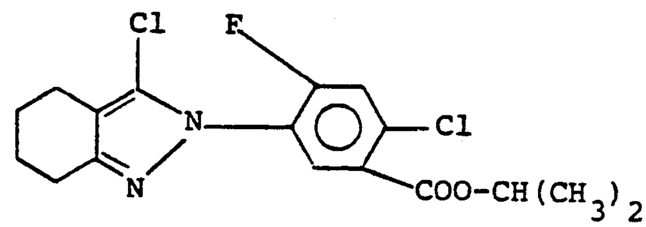

7. A method according to claim 1, wherein the compound of formula (I) is represented by the formula:

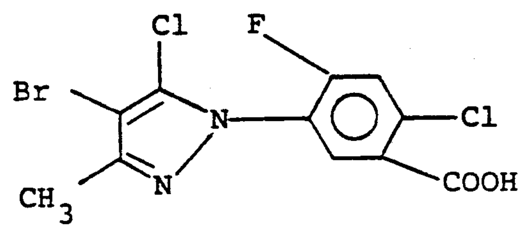

8. A process for the preparation of a compound of formula (I) as defined in claim 1 wherein $R^3$ represents hydroxy, ($C_3$ or $C_4$)-alkenyloxy, ($C_3$ or $C_4$)-alkynyloxy, ($C_1$ to $C_4$)-alkoxy-($C_1$ to $C_4$)-alkoxy, ($C_1$ to $C_4$)-alkylthio, ($C_1$ to $C_6$)-alkoxy unsubstituted or substituted by ($C_1$ to $C_4$)-alkoxycarbonyl; and $R^4$ represents halogen; which process comprises reacting a compound represented by the formula (II):

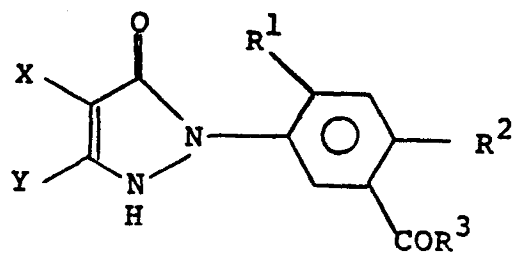

wherein $R^1$, $R^2$, X and Y are as defined in claim 1 and $R^3$ is as defined above, with a halogenating agent in an inert solvent.

9. A process for the preparation of a compound of formula (I) as defined in claim 1 wherein $R^1$, $R^2$, $R^3$, X and Y are as defined in claim 1 and $R^4$ is ($C_1$ to $C_4$)-alkoxy; which process comprises reacting a compound represented by the formula (II):

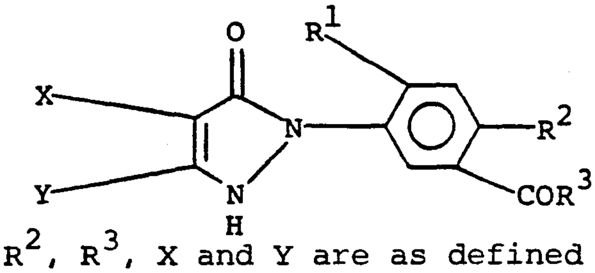

wherein $R^1$, $R^2$, $R^3$, X and Y are as defined above, with a compound represented by the formula (III):

$$(R^5O)_2SO_2$$

wherein $R^5$ is ($C_1$ to $C_4$)-alkyl, in an inert solvent and in the presence of a base.

10. A process for the preparation of a compound of formula (I) as defined in claim 1, wherein $R^1$, $R^2$, $R^3$, X and Y are as defined in claim 1 and $R^4$ is $-S(O)_m-R^5$ wherein m is 0, 1 or 2 and $R^5$ is a ($C_1$ to $C_4$)-alkyl group; which process comprises;

(1) heating a compound represented by the formula (XII):

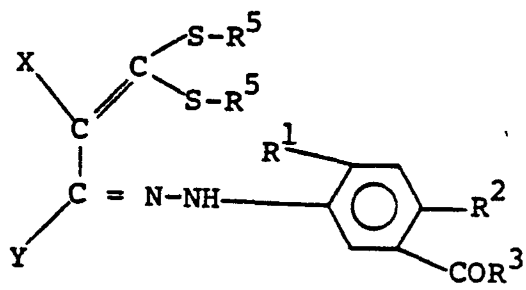

wherein $R^1$, $R^2$, $R^3$, $R^5$, X and Y are as defined above, in an inert solvent at a temperature up to the reflux temperature of the solvent, thereby obtaining a compound represented by the formula (XIII):

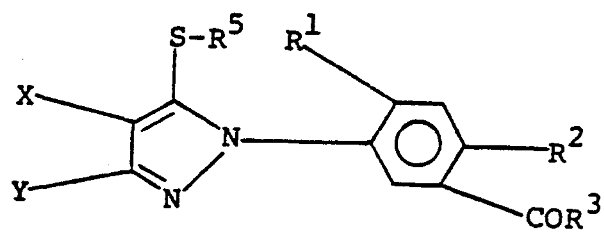

wherein $R^1$, $R^2$, $R^3$, $R^5$, X and Y are as defined above; and, if desired,

(2) reacting the compound of formula (XIII) thus-obtained with a peroxide in an inert solvent, thereby obtaining a compound represented by the formula (XIV):

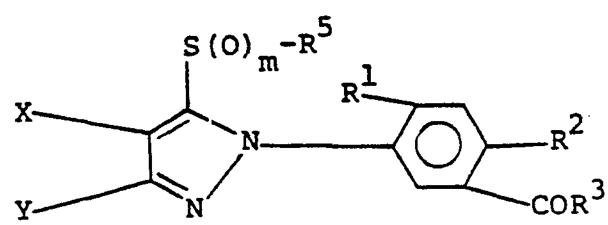

wherein $R^1$, $R^2$, $R^3$, $R^5$, X and Y are as defined above and m is 1 or 2.

11. A process for the preparation of a compound of formula (I) as defined in claim 1, which process comprises reacting a compound represented by the formula (XVI):

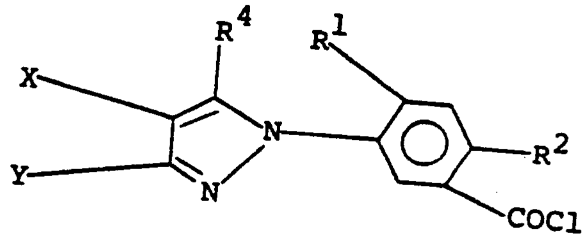

wherein $R^1$, $R^2$, $R^4$, X and Y are as defined in claim 1, with a compound represented by the formula (XVII):

$$H - R^3$$

wherein $R^3$ is as defined in claim 1, in an inert solvent.

12. A process for the preparation of a compound of formula (I) as defined in claim 1 wherein X is halogen; which process comprises reacting a compound represented by the formula (XVIII):

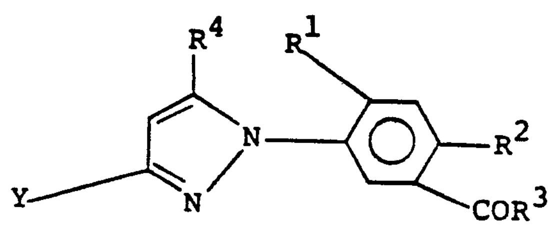

wherein $R^1$, $R^2$, $R^3$, $R^4$ and Y are as defined in claim 1, with chlorine, bromine or iodine in an inert solvent.

13. A process for the preparation of a compound of formula (I) as defined in claim 1 wherein $R^3$ is -OM wherein M is as defined in claim 1; which process comprises reacting a compound represented by the formula (XV):

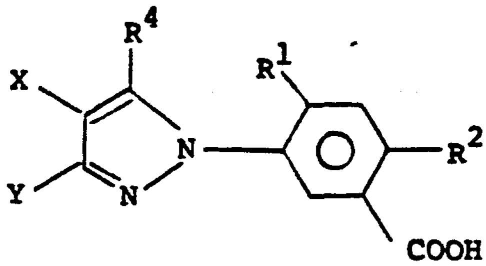

wherein $R^1$, $R^2$, $R^4$, X and Y are as defined in claim 1, with a salt of an alkali metal, an alkaline earth metal or ammonium or with a ($C_1$ to $C_4$)-alkylamine.